# EUROPEAN PATENT APPLICATION

(11) **EP 3 981 871 A1**
(43) Date of publication of application: **13.04.2022**
(21) Application number: 20823509.3
(22) Date of filing: 20.05.2020
(51) Int. Cl.: C12N 5/0775

(54) **COMPOSITION FOR PROMOTING PRODUCTION OF STEM CELL-DERIVED EXOSOMES AND INCREASING STEMNESS**

(30) Priority: 10.06.2019 KR 20190068042; 11.06.2019 KR 20190068915; 11.06.2019 KR 20190068929; 12.06.2019 KR 20190069585; 12.06.2019 KR 20190069582; 12.06.2019 KR 20190069586; 12.06.2019 KR 20190069590; 06.01.2020 KR 20200001518
(71) Applicant: Brexogen Inc., Seoul 05855 (KR)
(72) Inventor: KIM, Sue, Seoul 05649 (KR); LEE, Seulki, Gyeonggi-do 13390 (KR); KIM, Jimin, Seoul 05837 (KR)
(74) Representative: Plougmann Vingtoft a/s
(86) International application number: PCT/KR2020/006574
(87) International publication number: WO 2020/251181

(57) **Abstract**

The present disclosure relates to a composition for promoting the production of stem cell-derived exosomes or increasing the stemness of stem cells. When the composition of the present disclosure is used in culturing stem cells, the stemness of stem cells and the yield of stem cell-derived exosomes are increased, and thus good-quality stem cells and stem cell-derived exosomes can be produced more efficiently, and accordingly, can be advantageously used in related research and development and commercialization.

## Description

### Technical Field

The present disclosure relates to a composition for promoting the production of stem cell-derived exosomes and increasing stemness.

### Background Art

Extracellular vesicles are lipid bilayer-delimited vesicles particles including microvesicles, exosomes, and the like, which are spherical with a size of 30-1,000 nm.

Exosomes have lipid bilayers that are the same phospholipid bilayer structure as in source cells (donor cells), and are compositions of substances extracellularly excreted by cells, playing functional roles such as cell-cell communication and cellular immune intervention.

Exosomes carry cell-specific constituents accounting for biological functions characteristic of cells of origin and include various water-soluble proteins, peripheral proteins, and transmembrane proteins in addition to phospholipids, mRNA, and miRNA.

Such exosomes are released from all animal cells such as mast cells, lymphocytes, astrocytes, platelets, nerve cells, endothelial cells, epithelial cells, etc. and are found in various body fluids including blood, urine, mucus, saliva, bile juice, ascitic fluid, cerebrospinal fluid, and so on. Exhibiting high selective penetration sufficient to cross even the blood-brain barrier (BBB) as well as cell membranes of epidermal and endothelial cells, exosomes can find applications in the development of drug delivery systems utilizing nanocarriers for specific drugs.

Exosomes and microvesicles released from mesenchymal stem cells are involved in cell-to-cell communication and show medicinally regenerative therapeutic efficacy that stem cells possess.

Exosomes are known to bring about a trophic effect into paracrine factors secreted from stem cells that have been transplanted in vivo without survival for a long period of time. Released into such factors are small molecules such as growth factors, chemokines, cytokines, etc. by extracellular vesicles such as exosomes, and such exosomes are derived from stem cells. Therefore, exosomes are utilized for characterizing stem cells and evaluating therapeutic efficacies thereof.

In recent years, active research into therapeutic effects of exosomes secreted by mesenchymal stem cells, but not mesenchymal stem cells themselves, on various diseases have been ongoing. In the academia and the industries, it is expected that this strategy might be a new promising alternative that can surmount the limitation of conventional stem cell therapies.

For commercial applications, a large quantity of quality exosomes is needed. However, only a very small amount of exosomes can be currently obtained from stem cells and there has been still insufficient development of substances that can increase the production of stem cell-derived exosomes.

With the increase of the culture duration or the number of passages, mesenchymal stem cells decrease in the potential of proliferating and differentiating into various lineages. In addition, mesenchymal stem cells in a late passage remarkably decreases in colony forming efficacy, compared to those in an early passage. Senescence of mesenchymal stem cells seem to be associated with the attenuated proliferation activity due to long culture duration and also with a decrease in telomerase activity, rather than the age of the bone marrow donor.

As such, the amount of mesenchymal stem cells that can be obtained from humans is currently limited. In order to induce the differentiation of the cells thus obtained into a specific lineage, problems such as a decrease in differentiation capacity due to passages are inevitable. These drawbacks are impractical in inducing accurate differentiation into target cells and in clinical applications that require large amounts of appropriate stem cells for the differentiation induction. Therefore, there is an arising need for a method capable of maintaining the proliferation rate for a long period of time and maintaining the ability to differentiate into several lineages.

Accordingly, a need has arisen for the development of new methods and substances that can not only promote the production of stem cell-derived exosomes, but also maintain the proliferation rate and differentiation capacity of stem cells over a long period of time.

### Disclosure of Invention

### Technical Problem

Leading to the present disclosure, intensive and thorough research into the development of a method and a substance capable of maintaining the proliferation rate and differentiation capability of stem cells for a long period of time as well as promoting the production of stem cell-derived exosomes, conducted by the present inventors, resulted in the finding that pretreatment of stem cells with at least one selected from the group consisting of exendin-4, phorbol 12-myristate 13-acetate (PMA), interferon-γ, tetrandrine, hyaluronic acid, substance P, resveratrol, and lanifibranor not only improves stemness and proliferation in the stem cells, but also greatly increases the number of exosomes derived from the stem cells and the content of proteins and RNA in the exosomes.

Therefore, an aspect of the present disclosure is to provide a composition comprising exendin-4 for promoting the production of stem cell-derived exosomes.

Another aspect of the present disclosure is to provide a composition comprising exendin-4 for increasing stemness of stem cells.

Another aspect of the present disclosure is to provide a stem cell therapy product comprising the composition for promoting production of stem cell-derived exosomes or increasing stemness of stem cells.

Another aspect of the present disclosure is to provide stem cell-derived exosomes pretreated with exendin-4.

Another aspect of the present disclosure is to provide a stem cell therapy product comprising the stem cell-derived exosomes.

Another aspect of the present disclosure is to provide a method for producing stem cell-derived exosomes, the method comprising a pretreatment step of culturing stem cells in a cell culture medium containing exendin-4.

Another aspect of the present disclosure is to provide a method for increasing stemness of stem cells, the method comprising a pretreatment step of culturing stem cells in a cell culture medium containing exendin-4.

Another aspect of the present disclosure is to provide a use of a composition comprising exendin-4 in promoting production of stem cell-derived exosomes.

Another aspect of the present disclosure is to provide a use of a composition comprising exendin-4 in increasing stemness of stem cells.

Another aspect of the present disclosure is to provide a composition comprising phorbol 12-myristate 13-acetate (PMA) for promoting the production of stem cell-derived exosomes.

Another aspect of the present disclosure is to provide a composition comprising phorbol 12-myristate 13-acetate (PMA) for increasing stemness of stem cells.

Another aspect of the present disclosure is to provide a stem cell therapy product comprising the composition for promoting production of stem cell-derived exosomes or increasing stemness of stem cells.

Another aspect of the present disclosure is to provide stem cell-derived exosomes pretreated with phorbol 12-myristate 13-acetate (PMA).

Another aspect of the present disclosure is to provide a stem cell therapy product comprising the stem cell-derived exosomes.

Another aspect of the present disclosure is to provide a method for producing stem cell-derived exosomes, the method comprising a pretreatment step of culturing stem cells in a cell culture medium containing phorbol 12-myristate 13-acetate (PMA).

Another aspect of the present disclosure is to provide a method for increasing stemness of stem cells, the method comprising a pretreatment step of culturing stem cells in a cell culture medium containing phorbol 12-myristate 13-acetate (PMA).

Another aspect of the present disclosure is to provide a use of a composition comprising phorbol 12-myristate 13-acetate (PMA) in promoting production of stem cell-derived exosomes.

Another aspect of the present disclosure is to provide a use of a composition comprising phorbol 12-myristate 13-acetate (PMA) in increasing stemness of stem cells.

Another aspect of the present disclosure is to provide a composition comprising interferon-γ for promoting the production of stem cell-derived exosomes.

Another aspect of the present disclosure is to provide a composition comprising interferon-γ for increasing stemness of stem cells.

Another aspect of the present disclosure is to provide a stem cell therapy product comprising the composition for promoting production of stem cell-derived exosomes or increasing stemness of stem cells.

Another aspect of the present disclosure is to provide stem cell-derived exosomes pretreated with interferon-γ.

Another aspect of the present disclosure is to provide a stem cell therapy product comprising the stem cell-derived exosomes.

Another aspect of the present disclosure is to provide a method for producing stem cell-derived exosomes, the method comprising a pretreatment step of culturing stem cells in a cell culture medium containing interferon-γ.

Another aspect of the present disclosure is to provide a method for increasing stemness of stem cells, the method comprising a pretreatment step of culturing stem cells in a cell culture medium containing interferon-γ.

Another aspect of the present disclosure is to provide a use of a composition comprising interferon-γ in promoting production of stem cell-derived exosomes.

Another aspect of the present disclosure is to provide a use of a composition comprising interferon-γ in increasing stemness of stem cells.

Another aspect of the present disclosure is to provide a composition comprising tetrandrine for promoting the production of stem cell-derived exosomes.

Another aspect of the present disclosure is to provide a composition comprising tetrandrine for increasing stemness of stem cells.

Another aspect of the present disclosure is to provide a stem cell therapy product comprising the composition for promoting production of stem cell-derived exosomes or increasing stemness of stem cells.

Another aspect of the present disclosure is to provide stem cell-derived exosomes pretreated with tetrandrine.

Another aspect of the present disclosure is to provide a stem cell therapy product comprising the stem cell-derived exosomes.

Another aspect of the present disclosure is to provide a method for producing stem cell-derived exosomes, the method comprising a pretreatment step of culturing stem cells in a cell culture medium containing tetrandrine.

Another aspect of the present disclosure is to provide a method for increasing stemness of stem cells, the method comprising a pretreatment step of culturing stem cells in a cell culture medium containing tetrandrine.

Another aspect of the present disclosure is to provide a use of a composition comprising tetrandrine in promoting production of stem cell-derived exosomes.

Another aspect of the present disclosure is to provide a use of a composition comprising tetrandrine in increasing stemness of stem cells.

Another aspect of the present disclosure is to provide a composition comprising hyaluronic acid for promoting the production of stem cell-derived exosomes.

Another aspect of the present disclosure is to provide a composition comprising hyaluronic acid for increasing stemness of stem cells.

Another aspect of the present disclosure is to provide a stem cell therapy product comprising the composition for promoting production of stem cell-derived exosomes or increasing stemness of stem cells.

Another aspect of the present disclosure is to provide stem cell-derived exosomes pretreated with hyaluronic acid.

Another aspect of the present disclosure is to provide a stem cell therapy product comprising the stem cell-derived exosomes.

Another aspect of the present disclosure is to provide a method for producing stem cell-derived exosomes, the method comprising a pretreatment step of culturing stem cells in a cell culture medium containing hyaluronic acid.

Another aspect of the present disclosure is to provide a method for increasing stemness of stem cells, the method comprising a pretreatment step of culturing stem cells in a cell culture medium containing hyaluronic acid.

Another aspect of the present disclosure is to provide a use of a composition comprising hyaluronic acid in promoting production of stem cell-derived exosomes.

Another aspect of the present disclosure is to provide a use of a composition comprising hyaluronic acid in increasing stemness of stem cells.

Another aspect of the present disclosure is to provide a composition comprising substance P for promoting the production of stem cell-derived exosomes.

Another aspect of the present disclosure is to provide a composition comprising substance P for increasing stemness of stem cells.

Another aspect of the present disclosure is to provide a stem cell therapy product comprising the composition for promoting production of stem cell-derived exosomes or increasing stemness of stem cells.

Another aspect of the present disclosure is to provide stem cell-derived exosomes pretreated with substance P.

Another aspect of the present disclosure is to provide a stem cell therapy product comprising the stem cell-derived exosomes.

Another aspect of the present disclosure is to provide a method for producing stem cell-derived exosomes, the method comprising a pretreatment step of culturing stem cells in a cell culture medium containing substance P.

Another aspect of the present disclosure is to provide a method for increasing stemness of stem cells, the method comprising a pretreatment step of culturing stem cells in a cell culture medium containing substance P.

Another aspect of the present disclosure is to provide a use of a composition comprising substance P in promoting production of stem cell-derived exosomes.

Another aspect of the present disclosure is to provide a use of a composition comprising substance P in increasing stemness of stem cells.

Another aspect of the present disclosure is to provide a composition comprising resveratrol for promoting the production of stem cell-derived exosomes.

Another aspect of the present disclosure is to provide a composition comprising resveratrol for increasing stemness of stem cells.

Another aspect of the present disclosure is to provide a stem cell therapy product comprising the composition for promoting production of stem cell-derived exosomes or increasing stemness of stem cells. Another aspect of the present disclosure is to provide stem cell-derived exosomes pretreated with resveratrol.

Another aspect of the present disclosure is to provide a stem cell therapy product comprising the stem cell-derived exosomes.

Another aspect of the present disclosure is to provide a method for producing stem cell-derived exosomes, the method comprising a pretreatment step of culturing stem cells in a cell culture medium containing resveratrol.

Another aspect of the present disclosure is to provide a method for increasing stemness of stem cells, the method comprising a pretreatment step of culturing stem cells in a cell culture medium containing resveratrol.

Another aspect of the present disclosure is to provide a use of a composition comprising resveratrol in promoting production of stem cell-derived exosomes.

Another aspect of the present disclosure is to provide a use of a composition comprising resveratrol in increasing stemness of stem cells.

Another aspect of the present disclosure is to provide a composition comprising lanifibranor for promoting the production of stem cell-derived exosomes.

Another aspect of the present disclosure is to provide a stem cell therapy product comprising lanifibranor for promoting the production of stem cell-derived exosomes.

Another aspect of the present disclosure is to provide a composition comprising lanifibranor for increasing stemness of stem cells.

Another aspect of the present disclosure is to provide a stem cell therapy product comprising lanifibranor for reinforcing stemness of stem cells. Another aspect of the present disclosure is to provide stem cell-derived exosomes pretreated with lanifibranor.

Another aspect of the present disclosure is to provide a stem cell therapy product comprising the stem cell-derived exosomes pretreated with lanifibranor as an active ingredient.

Another aspect of the present disclosure is to provide a method for producing stem cell-derived exosomes, the method comprising a pretreatment step of culturing stem cells in a cell culture medium containing lanifibranor.

Another aspect of the present disclosure is to provide a method for increasing stemness of stem cells, the method comprising a pretreatment step of culturing stem cells in a cell culture medium containing lanifibranor.

Another aspect of the present disclosure is to provide a use of a composition comprising lanifibranor in promoting production of stem cell-derived exosomes.

Another aspect of the present disclosure is to provide a use of a composition comprising lanifibranor in increasing stemness of stem cells.

### Solution to Problem

The present inventors have made effects to development a method and a substance capable of maintaining the proliferation rate and differentiation capability of stem cells for a long period of time as well as promoting the production of stem cell-derived exosomes. As a consequence, it was found that when stem cells are pretreated with at least one selected from the group consisting of exendin-4, phorbol 12-myristate 13-acetate (PMA), interferon-γ, tetrandrine, hyaluronic acid, substance P, resveratrol, and lanifibranor, not only do the stem cells improve in stemness and proliferation, but also the number of exosomes derived from the stem cells and the content of proteins and RNA in the exosomes are greatly increased.

The present disclosure pertains to: a composition for promoting production of stem cell-derived exosomes or for increasing stemness of stem cells, the composition comprising at least one selected from the group consisting of exendin-4, phorbol 12-myristate 13-acetate (PMA), interferon-γ, tetrandrine, hyaluronic acid, substance P, resveratrol, and lanifibranor; a stem cell therapy product comprising the composition; an exosome derived from a stem cell pretreated with at least one selected from the group consisting of exendin-4, phorbol 12-myristate 13-acetate (PMA), interferon-γ, tetrandrine, hyaluronic acid, substance P, resveratrol, and lanifibranor; a stem cell therapy product comprising the exosome derived from the stem cell; a method for production of a stem cell-derived exosome; and a method for increasing stemness of a stem cell.

Below, a detailed description will be given of the present disclosure.

An aspect of the present disclosure contemplates a composition comprising exendin-4 for promoting production of a stem cell-derived exosome.

Exendin-4 used in the present disclosure is a peptide agonist of the glucagon-like peptide (GLP) receptor. Known to promote insulin secretion, exendin-4 has been clinically used for type-2 diabetes mellitus and Parkinson's disease.

An aspect of the present disclosure contemplates a composition comprising phorbol 12-myristate 13-acetate (PMA) for promoting production of a stem cell-derived exosome.

Phorbol 12-myristate 13-acetate (PMA) used in the present disclosure, also known as 12-0-tetradecanoylphorbol-13-acetate (TPA), acts to activate the signal transduction enzyme protein kinase C (PKC). In addition, PMA is used as a tumor promoter and to stimulate division of B cells, etc. PMA also induces the activation of many cell types to produce cytokines. Accordingly, PMA has been employed in biomedical research to evoke inflammatory reactions.

An aspect of the present disclosure contemplates a composition comprising interferon-γ for promoting production of a stem cell-derived exosome.

Interferon-γ used in the present disclosure is a cytokine that is produced by activated T cells or NK cells, and CD4- or CD8-positive lymphocytes. Serving as a differentiation stimulator or growth factor, the cytokine is involved in the activation and proliferative differentiation of various cells including T lymphocytes and in the action of increasing expression of MHC on antigen-presenting cells and plays a critical role in immune and inflammatory responses. Moreover, interferon-γ has been clinically used for therapy of infection and autoimmune disease.

An aspect of the present disclosure contemplates a composition comprising tetrandrine for promoting production of a stem cell-derived exosome.

Tetrandrine used in the present disclosure, which is a calcium channel broker, non-selectively inhibits calcium channel activity and induces cell cycle arrest at G1 phase in various types of cells, thus exhibiting immunosuppressive, anti-inflammatory, antifibrotic, and anticoagulatory activities. Also, tetrandrine enhances glycogenesis in hepatocytes to increase glucose utilization, resulting in the lowering of plasma glucose. Furthermore, tetrandrine has been clinically used as a co-therapeutic agent for diabetes mellitus and for therapy of hypertension, arrhythmia, pain and convulsion, liver cirrhosis, and rheumatoid arthritis.

An aspect of the present disclosure contemplates a composition comprising hyaluronic acid for promoting production of a stem cell-derived exosome.

Hyaluronic acid used in the present disclosure is a natural linear polysaccharide having disaccharide units composed of D-glucuronic acid and N-acetyl-D-glucosamine, linked via a β(1→4) inter-glycosidic linkage. Hyaluronic acid, known as a major component of the dermis, is a biomaterial excellent in biocompatibility and biodegradability and free of in vivo immune rejection responses and toxicity. As a highly viscous and elastic natural glycosaminoglycan macromolecule with various functions and physicochemical properties, hyaluronic acid has found clinical applications in arthritis therapy, ophthalmic surgery, wound healing, and tissue engineering and as a filler, a drug carrier, an anti-adhesion barrier, and a cosmetic.

An aspect of the present disclosure contemplates a composition comprising substance P for promoting production of a stem cell-derived exosome.

Substance P used in the present disclosure is an undecapeptide member of the tachykinin neuropeptide family. It is a neuropeptide, acting as a neurotransmitter and as a neuromodulator on NK1 receptor. In addition, substance P is a potent vasodilator and is excitatory to cell growth. With such activities, substance P is known to promote would healing of non-healing ulcers as well as exhibiting therapeutic effects on chronic hives, atopic dermatitis, and chronic renal disease and regulatory effects on inflammation and pain. Furthermore, substance P is helpful in proliferating and regenerating skin cells, thus finding applications as a cosmetic material and in the clinical therapy of hypertension and liver cirrhosis disease.

An aspect of the present disclosure contemplates a composition comprising resveratrol for promoting production of a stem cell-derived exosome.

Resveratrol used in the present disclosure is a phytoalexin, a type of polyphenols, which is produced by plants when the plants are under attack by fungi or insects. Resveratrol activates the deacetylase Sirt1 (sirtuin-1) to enhance mitochondrial functions and the flow of sugar metabolism. In addition, resveratrol has evidences of anti-disease effects including anticancer, antiviral, neuroprotective, anti-aging, anti-inflammatory, and life-extension effects. Also, resveratrol shows antioxidative activity, and stimulates nitrogen oxide (NO) production to dilate vessels, resulting in protection against reperfusion injury and inhibitory effect on arrhythmia. Accordingly, resveratrol has been continually studied for therapeutic use in cardiovascular and brain diseases and found clinical applications in various fields including antiaging, anticancer-related health function foods, anti-wrinkling cosmetics.

An aspect of the present disclosure contemplates a composition comprising lanifibranor for promoting production of a stem cell-derived exosome.

Lanifibranor used in the present disclosure, which is a peroxisome proliferator-activated receptor (PPAR) agonist, is a small molecule that acts to induce anti-fibric, anti-inflammatory as well as beneficial metabolic changes in the body by activating each of the three PPAR isoforms, known as PPARα, PPARδ, and PPARγ. PPARs are a family of nuclear hormone receptors that function as ligand-activated transcription factors regulating the expression of genes. PPARs play essential roles in the regulation of cellular differentiation, development, and tumorigenesis. By activating PPARs regulatory of fibrosis, lanifibranor is known to reduce abnormal growth of connective tissues. In addition, lanifibranor has been clinically used as a therapeutic agent for systemic sclerosis, idiopathic pulmonary fibrosis, etc.

The composition according to the present disclosure may further comprise an exosome production stimulating substance in addition to the ingredients described above.

As used herein, the term "exosome" refers to a cell-derived vesicle. Exosomes are found in body fluids of almost all eukaryotic organisms. Exosomes are about 30-200 nm in size, larger than LDL proteins, but far smaller than erythrocytes.

When multivesicular bodies fuse to cell membranes, exosomes are secreted from cells or just from the cell membranes.

Such exosomes are well known to perform important and specialized functions such as coagulation, cell-to-cell signaling, etc.

As used herein, the term "stem cell" refers to an undifferentiated cell that can self-renew and differentiate into two or more different types of cells.

No limitations are imparted to the stem cells. For example, the stem cells may be autogenous or homogenous stem cells, may be originated from any type of animals including humans and non-human mammals, and may be derived from adults or embryos.

In detail, the stem cells may be embryonic stem cells, adult stem cells, or induced pluripotent stem cells, but with no limitations thereto.

As used herein, the term "embryonic stem cell" refers to a cell isolated during the embryogenesis, which is derived from the inner cell mass of a blastocyst formed prior to implantation of the fertilized egg in the uterus, and cultured in vitro.

Embryonic stem cells are cells that are pluripotent or totipotent and give rise to differentiation to cells of all tissues and exhibit self-renewal, and encompass embryoid bodies derived therefrom in a wide sense.

The embryonic stem cells may be derived from any source such as humans, monkeys, pigs, horses, cattle, sheep, dogs, cats, rabbits, etc., but with no limitations thereto.

As used herein, the term "adult stem cell" refers to an undifferentiated cell just before differentiation to cells of specific organs, found in umbilical cord blood, adult's bone marrow, blood, etc., which has the ability to develop to tissues of the body as needed.

The adult stem cells are intended to encompass adult stem cells from various tissues of human or animal origin, mesenchymal stromal cells of human or animal origin, and mesenchymal stromal cells and multipotent stem cells derived from induced pluripotent stem cells from various tissues of human or animal origin, but with no limitations thereto.

The human or animal tissues may include, but are not limited to umbilical cord, umbilical cord blood, lipid, muscle, nerve, skin, amnion, and/or placenta.

The stem cells from various tissues of human or animal origin may be hematopoietic stem cells, mammary stem cells, intestinal stem cells, vascular endothelial progenitor cells, neural stem cells, olfactory neural stem cell, and/or testicular stem cells, but are not limited thereto.

As used herein, the term "induced pluripotent stem cell" (iPSC) means pluripotent cells derived from differentiated cells through artificial dedifferentiation and may be interchangeably used with "dedifferentiated stem cell".

The artificial dedifferentiation may be conducted by introducing dedifferentiation factors through viral mediation using retroviruses, lentiviruses, and sendai viruses, through non-viral mediation with the aid of non-viral vectors, proteins, and cell extracts, or through stem cell extracts, compounds, etc.

Induced pluripotent stem cells exhibit almost the same characteristics as embryonic stem cells. In detail, they are common in the aspects including cellular morphological similarity, similar gene and protein expression pattern, totipotency in vitro and in vivo, teratoma formation, creation of chimeric mice upon insertion into murine blastocysts, and germline transmission of genes.

The induced pluripotent stem cells may include those of any origin, such as humans, monkeys, pigs, horses, cattle, sheep, cats, mice, rabbits, etc., but with no limitations thereto.

As used herein, the term "promoting production" refers to increasing the production, generation, and release of a specific substance within the same time, compared to a control.

In detail, the term is intended to encompass an increased quantity of exosomes secreted from stem cells and an increase content of proteins, RNA, etc. within the exosomes.

Another aspect of the present disclosure pertains to a composition comprising exendin-4 for increasing stemness of stem cells.

Another aspect of the present disclosure pertains to a composition comprising phorbol 12-myristate 13-acetate (PMA) for increasing stemness of stem cells.

Another aspect of the present disclosure pertains to a composition comprising interferon-γ for increasing stemness of stem cells.

Another aspect of the present disclosure pertains to a composition comprising tetrandrine for increasing stemness of stem cells.

Another aspect of the present disclosure pertains to a composition comprising hyaluronic acid for increasing stemness of stem cells.

Another aspect of the present disclosure pertains to a composition comprising substance P for increasing stemness of stem cells.

Another aspect of the present disclosure pertains to a composition comprising resveratrol for increasing stemness of stem cells.

Another aspect of the present disclosure pertains to a composition comprising lanifibranor for increasing stemness of stem cells.

The term "sternness", as used herein, is used in the art to encompass the capability of a cell for pluripotency of differentiating into all types of cells and for self-renewal of dividing indefinitely to produce more of the same stem cell.

In detail, stemness accounts for at least one of the capabilities for increasing proliferation of stem cells while maintaining stem cells in an undifferentiated state, for increasing telomerase activity, for increasing expression of stemness acting signals, and for increasing cell mobility.

Each of the compositions of the present disclosure may be used as a medium composition in culturing stem cells or may be administered as a pharmaceutical composition for promoting in vivo production of exosomes to the body, together with, prior to, or subsequently to stem cells, so as to reinforce in vivo efficacy of stem cells.

As used herein, the term "reinforce in vivo efficacy of stem cells" means that under the assumption that the efficacy (in vivo efficacy) of stem cells for therapy of a specific disease such as arthritis, neuropathy, etc. is mediated by exosomes secreted by the stem cells, the co-administration of the composition of the present disclosure and the stem cells promotes the production of exosomes from the stem cells, resulting in reinforcing the therapeutic effect (in vivo efficacy) of the stem cells on the disease.

In the context of using the each of the compositions of the present disclosure as a medium composition, the term "medium composition" means a composition containing essential ingredients necessary for cell growth, survival, and proliferation in vitro and encompasses all media typically used in the art for culturing stem cells in vitro. For example, commercially available media such as DMEM(Dulbecco's Modified Eagle's Medium), MEM (Minimal Essential Medium), BME(Basal Medium Eagle), RPMI 1640, DMEM/F-10 (Dulbecco's Modified Eagle's Medium: Nutrient Mixture F-10), DMEM/F-12 (Dulbecco's Modified Eagle's Medium: Nutrient Mixture F-12), α-MEM(α-Minimal essential Medium), G-MEM(Glasgow's Minimal Essential Medium), IMDM(Isocove's Modified Dulbecco's Medium), KnockOut DMEM, and E8 (Essential 8 Medium), or artificially synthetic media may be included, but with no limitations thereto.

The medium composition may comprise a carbon source, a nitrogen source, and a trace element ingredient and additionally an amino acid, an antibiotic, etc.

Also, the medium composition may be prepared by adding the ingredient exendin-4 of the present disclosure to a conventional stem cell culturing medium. Also, the medium composition may be prepared by adding the ingredient phorbol 12-myristate 13-acetate (PMA) of the present disclosure to a conventional stem cell culturing medium.

Also, the medium composition may be prepared by adding the ingredient interferon-γ of the present disclosure to a conventional stem cell culturing medium.

Also, the medium composition may be prepared by adding the ingredient tetrandrine of the present disclosure to a conventional stem cell culturing medium.

Also, the medium composition may be prepared by adding the ingredient hyaluronic acid of the present disclosure to a conventional stem cell culturing medium.

Also, the medium composition may be prepared by adding the ingredient substance P of the present disclosure to a conventional stem cell culturing medium.

Also, the medium composition may be prepared by adding the ingredient resveratrol of the present disclosure to a conventional stem cell culturing medium.

Also, the medium composition may be prepared by adding the ingredient lanifibranor of the present disclosure to a conventional stem cell culturing medium.

The ingredient exendin-4 of the present disclosure may be added at a concentration of 10-1000 nM, 10-100 nM, 10-90 nM, 10-80 nM, 10-70 nM, 10-60 nM, 10-50 nM, 10-40 nM, 10-30 nM, or 20 nM to the medium, but with no limitations thereto.

The ingredient phorbol 12-myristate 13-acetate (PMA) of the present disclosure may be added at a concentration of 1-1000 nM, 1-500 nM, 1-100 nM, 1-90 nM, 1-80 nM, 1-70 nM, 1-60 nM, 1-50 nM, 1-40 nM, 1-30 nM, 1-20 nM, or 10 nM to the medium, but with no limitations thereto.

The ingredient interferon-γ of the present disclosure may be added at a concentration of 1-1000 ng/mL, 1-500 ng/mL, 1-100 ng/mL, 1-90 ng/mL, 1-80 ng/mL, 1-70 ng/mL, 1-60 ng/mL, 1-50 ng/mL, 1-40 ng/mL, 1-30 ng/mL, 1-20 ng/mL, or 10 ng/mL to the medium, but with no limitations thereto.

The ingredient tetrandrine of the present disclosure may be added at a concentration of 1-1000 µM, 1-500 µM, 1-100 µM, 1-90 µM, 1-80 µM, 1-70 µM, 1-60 µM, 1-50 µM, 1-40 µM, 1-30 µM, 1-20 µM, or 10 µM to the medium, but with no limitations thereto.

The ingredient hyaluronic acid of the present disclosure may be added at a concentration of 1-1000 µg/mL, 1-500 µg/mL, 1-100 µg/mL, 1-90 µg/mL, 1-80 µg/mL, 1-70 µg/mL, 1-60 µg/mL, 1-50 µg/mL, 1-40 µg/mL, 1-30 µg/mL, 1-20 µg/mL, or 10 µg/mL to the medium, but with no limitations thereto.

The ingredient substance P of the present disclosure may be added at a concentration of 10-1000 nM, 10-100 nM, 10-90 nM, 10-80 nM, 10-70 nM, 10-60 nM, 10-50 nM, 10-40 nM, 20-40 nM, or 30 nM to the medium, but with no limitations thereto.

The ingredient resveratrol of the present disclosure may be added at a concentration of 1-1000 nM, 1-500 nM, 1-100 nM, 1-90 nM, 1-80 nM, 1-70 nM, 1-60 nM, 1-50 nM, 1-40 nM, 1-30 nM, 1-20 nM, or 10 nM to the medium, but with no limitations thereto.

The ingredient lanifibranor of the present disclosure may be added at a concentration of 1 -1000 µM, 1-500 µM, 1-100 µM, 1-90 µM, 1-80 µM, 1-70 µM, 1-60 µM, 1-50 µM, 1-40 µM, 1-30 µM, 1-20 µM, or 10 µM to the medium, but with no limitations thereto.

In cases where each of the compositions of the present disclosure is prepared into a pharmaceutical composition, the pharmaceutical composition of the present disclosure comprises a pharmaceutically acceptable carrier. The pharmaceutically acceptable carrier contained in the pharmaceutical composition of the present disclosure is the same as in ordinary formulations, and examples thereof may include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like. The pharmaceutical composition of the present disclosure may further comprise, in addition to the above ingredients, a lubricant, a humectant, a sweetener, a flavoring agent, an emulsifier, a suspending agent, a preservative, and the like. For details of suitable pharmaceutically acceptable carriers and preparations, reference may be made to Remington's Pharmaceutical Sciences (19th ed., 1995).

The pharmaceutical composition of the present disclosure may be administered orally or parenterally, and for example, via intravenous, subcutaneous, intramuscular, intraperitoneal, topical, intranasal, intrapulmonary, rectal, intrathecal, intraocular, percutaneous, and transdermal routes.

A suitable dose of the pharmaceutical composition of the present disclosure may be vary depending on various factors including the method of formulation, the manner of administration, patient's age, body weight, sex, disease state, food, time of administration, route of administration, excretion rate, and response sensitivity. A skilled physician can easily determine and prescribe a dose effective for desired therapy or prophylaxis. According to an embodiment of the present disclosure, a daily dose of the pharmaceutical composition of the present disclosure is within a range of 0.0001-1000 mg/kg.

The pharmaceutical composition of the present disclosure may be formulated using a pharmaceutically acceptable carrier and/or excipient according to a method that could be easily performed by a person having ordinary skills in the art to which the present disclosure pertains, and the composition of the present disclosure may be prepared into a unit dosage form or may be contained in a multi-dose container. Here, the formulation may be in the form of a solution in an oily or aqueous medium, a suspension, an emulsion, an extract, a pulvis, a suppository, a powder, granules, a tablet, or a capsule, and may further comprise a dispersant or a stabilizer.

Another aspect of the present disclosure is concerned with stem cell-derived exosomes pretreated with exendin-4.

Another aspect of the present disclosure is concerned with stem cell-derived exosomes pretreated with phorbol 12-myristate 13-acetate (PMA).

Another aspect of the present disclosure is concerned with stem cell-derived exosomes pretreated with interferon-γ.

Another aspect of the present disclosure is concerned with stem cell-derived exosomes pretreated with tetrandrine.

Another aspect of the present disclosure is concerned with stem cell-derived exosomes pretreated with hyaluronic acid.

Another aspect of the present disclosure is concerned with stem cell-derived exosomes pretreated with substance P.

Another aspect of the present disclosure is concerned with stem cell-derived exosomes pretreated with resveratrol.

Another aspect of the present disclosure is concerned with stem cell-derived exosomes pretreated with lanifibranor.

The common content between each of the compositions of the present disclosure and the stem cell-derived exosomes will be omitted in order to avoid undue complexity of the specification.

Another aspect of the present disclosure is concerned with a stem cell therapy product comprising the composition comprising exendin-4, the stem cell pretreated with exendin-4, or the stem cell-derived exosomes pretreated with exendin-4.

Another aspect of the present disclosure is concerned with a stem cell therapy product comprising the composition comprising phorbol 12-myristate 13-acetate (PMA), the stem cell pretreated with phorbol 12-myristate 13-acetate (PMA), or the stem cell-derived exosomes pretreated with phorbol 12-myristate 13-acetate (PMA) .

Another aspect of the present disclosure is concerned with a stem cell therapy product comprising the composition comprising interferon-γ, the stem cell pretreated with interferon-γ, or the stem cell-derived exosomes pretreated with interferon-γ.

Another aspect of the present disclosure is concerned with a stem cell therapy product comprising the composition comprising tetrandrine, the stem cell pretreated with tetrandrine, or the stem cell-derived exosomes pretreated with tetrandrine.

Another aspect of the present disclosure is concerned with a stem cell therapy product comprising the composition comprising hyaluronic acid, the stem cell pretreated with hyaluronic acid, or the stem cell-derived exosomes pretreated with hyaluronic acid.

Another aspect of the present disclosure is concerned with a stem cell therapy product comprising the composition comprising substance P, the stem cell pretreated with substance P, or the stem cell-derived exosomes pretreated with substance P.

Another aspect of the present disclosure is concerned with a stem cell therapy product comprising the composition comprising resveratrol, the stem cell pretreated with resveratrol, or the stem cell-derived exosomes pretreated with resveratrol.

Another aspect of the present disclosure is concerned with a stem cell therapy product comprising the composition comprising lanifibranor, the stem cell pretreated with lanifibranor, or the stem cell-derived exosomes pretreated with lanifibranor.

As used herein, the term "cell therapy product" means a medicinal product used for the purpose of therapy, diagnosis, and prophylaxis through a series of actions including proliferating and selecting autologous, allogeneic, or xenogeneic cells in vitro, changing biological properties of cells by other methods so as to restore functions of cells and tissues.

Such cell therapy products are largely divided into two types, including "stem cell therapy products" for tissue regeneration, organ function restoration, or modulation of immune cell functions, and "immune cell therapy products" for immunomodulation such as suppression or enhancement of immune responses in vivo.

Herein, "cell therapy product" refers to "stem cell therapy product" because the individual compositions and their ingredients exendin-4, phorbol 12-myristate 13-acetate (PMA), interferon-γ, tetrandrine, hyaluronic acid, substance P, resveratrol, and lanifibranor according to the present disclosure increase stemness of stem cells and promote the production of stem cell-derived exosomes with the consequent reinforcement of therapeutic efficacy of stem cells.

The stem cell therapy product may be used to treat a disease in a subject in need thereof, examples of which include cardiovascular diseases such as myocardial infarction, heart failure, and so on, cancer diseases such as liver cancer, stomach cancer, colorectal cancer, prostate cancer, bladder cancer, lung cancer, and so on,, inflammatory diseases such as atopic dermatitis, arthritis, autoimmune encephalomyelitis, systemic lupus erythematosus, colitis, and multiple sclerosis, and autoimmune disease, but are not limited thereto.

The stem cell therapy product has ingredients in common with each of the compositions described above according to the present disclosure, and the common content therebetween will be omitted in order to avoid undue complexity of the specification.

Another aspect of the present disclosure relates to a method for producing stem cell-derived exosomes, the method comprising a step of: a) culturing stem cells in a cell culture medium containing exendin-4.

Another aspect of the present disclosure relates to a method for producing stem cell-derived exosomes, the method comprising a step of: a) culturing stem cells in a cell culture medium containing phorbol 12-myristate 13-acetate (PMA).

Another aspect of the present disclosure relates to a method for producing stem cell-derived exosomes, the method comprising a step of: a) culturing stem cells in a cell culture medium containing a) interferon-γ.

Another aspect of the present disclosure relates to a method for producing stem cell-derived exosomes, the method comprising a step of: a) culturing stem cells in a cell culture medium containing tetrandrine.

Another aspect of the present disclosure relates to a method for producing stem cell-derived exosomes, the method comprising a step of: a) culturing stem cells in a cell culture medium containing hyaluronic acid.

Another aspect of the present disclosure relates to a method for producing stem cell-derived exosomes, the method comprising a step of: a) culturing stem cells in a cell culture medium containing substance P.

Another aspect of the present disclosure relates to a method for producing stem cell-derived exosomes, the method comprising a step of: a) culturing stem cells in a cell culture medium containing resveratrol.

Another aspect of the present disclosure relates to a method for producing stem cell-derived exosomes, the method comprising a step of: a) culturing stem cells in a cell culture medium containing lanifibranor.

Each of the method may further comprising the steps of:
b) washing the cultured stem cells, followed by additional culturing in a cell culture medium; and
c) isolating exosomes.

Hereinafter, the methods for producing stem cell-derived exosomes of the present disclosure will be described in detail.

### Step a)

This step is a process of stimulating stem cells by pretreatment with exendin-4, phorbol 12-myristate 13-acetate (PMA), interferon-γ, tetrandrine, hyaluronic acid, substance P, resveratrol, or lanifibranor. In this process, the stem cells produce more numbers of exosomes and increases contents of proteins and RNA within the exosomes, compared to stem cells which have not been treated with any of the substances.

### Step b)

This step is a process in which the stem cells pretreated with the substances are washed to remove the pretreatment substances and are cultured in a new cell culture medium to induce secretion or production of exosomes from the stem cells. The pretreatment substance (e.g., exending-4) in step a) is removed by the washing process of step b) and is not left in the stem cells, exosomes produced by the stem cells, or the culture medium. Therefore, the stem cells pretreated with the pretreatment substance of the present disclosure, exosomes produced from the stem cells, and the culture medium can be advantageously used for subsequent studies or in therapy for diseases without influence of residual pretreatment substances.

In this step, the cell culture medium may additionally contain exosome-free fetal bovine serum (FBS). Exosome-free FBS is required in the cell culture medium in order to prevent incorporation of other exosomes because generally used FBS contain a large amount of exosomes derived from bovine serum.

The additional culturing in this step may be conducted for 12 to 120 hours, 24 to 96 hours, 48 to 96 hours, or 60 to 84 hours, and most particularly for 72 hours, but with no limitations thereto.

### Step c)

In this step, the culture medium in which the stem cells have been additional cultured in step b) is centrifuged at 200-400xg for 5 to 20 min. After removal of the cell pellet and cell debris, the supernatant was subjected to high-speed centrifugation at 9,000-12,000xg for 60-80 min. The supernatant thus obtained was subjected again to ultracentrifugation at 90,000-120,000xg for 80-100 min. Exosomes were obtained as a pellet by removing the supernatant.

According to an embodiment of the present disclosure, a culture medium in which mesenchymal stromal stem cells have been cultured is taken and centrifuged at 300xg for 10 min. After the cell pellet and cell debris are removed, the supernatant was filtered through a 0.22 µm filter and the filtrate was centrifuged at 10,000xg and 4°C for 70 min using a high-speed centrifuge. The supernatant was taken and subjected again to ultracentrifugation at 100,000xg and 4°C for 90 min. Exosomes were isolated as a pellet.

Another aspect of the present disclosure relates to a method for increasing stemness of stem cells, the method comprising a step of: a) culturing stem cells in a cell culture medium containing exendin-4.

Another aspect of the present disclosure relates to a method for increasing stemness of stem cells, the method comprising a step of: a) culturing stem cells in a cell culture medium containing phorbol 12-myristate 13-acetate (PMA).

Another aspect of the present disclosure relates to a method for increasing stemness of stem cells, the method comprising a step of: a) culturing stem cells in a cell culture medium containing interferon-γ.

Another aspect of the present disclosure relates to a method for increasing stemness of stem cells, the method comprising a step of: a) culturing stem cells in a cell culture medium containing tetrandrine.

Another aspect of the present disclosure relates to a method for increasing stemness of stem cells, the method comprising a step of: a) culturing stem cells in a cell culture medium containing hyaluronic acid.

Another aspect of the present disclosure relates to a method for increasing stemness of stem cells, the method comprising a step of: a) culturing stem cells in a cell culture medium containing substance P.

Another aspect of the present disclosure relates to a method for increasing stemness of stem cells, the method comprising a step of: a) culturing stem cells in a cell culture medium containing resveratrol.

Another aspect of the present disclosure relates to a method for increasing stemness of stem cells, the method comprising a step of: a) culturing stem cells in a cell culture medium containing lanifibranor.

The method for increasing stemness of stem cells has steps in common with the method for producing stem cell-derived exosomes described above according to the present disclosure, and the common content therebetween will be omitted in order to avoid undue complexity of the specification.

According to another aspect thereof, the present disclosure provides a use of a composition comprising exendin-4 in promoting production of stem cell-derived exosomes.

According to another aspect thereof, the present disclosure provides a use of a composition comprising exendin-4 in increasing stemness of stem cells.

According to another aspect thereof, the present disclosure provides a use of a composition comprising phorbol 12-myristate 13-acetate (PMA) in promoting production of stem cell-derived exosomes.

According to another aspect thereof, the present disclosure provides a use of a composition comprising phorbol 12-myristate 13-acetate (PMA) in increasing stemness of stem cells.

According to another aspect thereof, the present disclosure provides a use of a composition comprising interferon-γ in promoting production of stem cell-derived exosomes.

According to another aspect thereof, the present disclosure provides a use of a composition comprising interferon-γ in increasing stemness of stem cells.

According to another aspect thereof, the present disclosure provides a use of a composition comprising tetrandrine in promoting production of stem cell-derived exosomes.

According to another aspect thereof, the present disclosure provides a use of a composition comprising tetrandrine in increasing stemness of stem cells.

According to another aspect thereof, the present disclosure provides a use of a composition comprising hyaluronic acid in promoting production of stem cell-derived exosomes.

According to another aspect thereof, the present disclosure provides a use of a composition comprising hyaluronic acid in increasing stemness of stem cells.

According to another aspect thereof, the present disclosure provides a use of a composition comprising substance P in promoting production of stem cell-derived exosomes.

According to another aspect thereof, the present disclosure provides a use of a composition comprising substance P in increasing stemness of stem cells.

According to another aspect thereof, the present disclosure provides a use of a composition comprising resveratrol in promoting production of stem cell-derived exosomes.

According to another aspect thereof, the present disclosure provides a use of a composition comprising resveratrol in increasing stemness of stem cells.

According to another aspect thereof, the present disclosure provides a use of a composition comprising lanifibranor in promoting production of stem cell-derived exosomes.

According to another aspect thereof, the present disclosure provides a use of a composition comprising lanifibranor in increasing stemness of stem cells.

### Advantageous Effects of Invention

The present disclosure pertains to a composition for promoting the production of stem cell-derived exosomes and reinforcing the functions of stem cells, the composition comprising at least one selected from among exendin-4, phorbol 12-myristate 13-acetate (PMA), interferon-γ, tetrandrine, hyaluronic acid, substance P, resveratrol, and lanifibranor. When used in culturing stem cells, the composition of the present disclosure increases the stemness of stem cells and the productivity of stem cell-derived exosomes to make effective production of quality stem cells and stem cell-derived exosomes, thus finding advantageous applications in the research and development and commercialization of stem cells and stem cell-derived exosomes.

### Brief Description of Drawings

FIG. 1 is a graph illustrating an increase of stem cells in proliferation with the treatment of the stem cells with a stem cell pretreatment substance (exendin-4) according to an embodiment of the present disclosure.
FIGS. 2a and 2b are views illustrating an increase of stem cells in stemness with the treatment of the stem cells with stem cell pretreatment substance (exendin-4) according to an embodiment of the present disclosure.
FIG. 3 is a size distribution plot of exosomes after treatment with the stem cell pretreatment substance (exendin-4) according to an embodiment of the present disclosure.
FIG. 4 is a graph illustrating an increase in the number of exosomes with the treatment of the stem cells with a stem cell pretreatment substance (exendin-4) according to an embodiment of the present disclosure.
FIG. 5 is a graph illustrating an increase in the amount of exosomal proteins with the treatment of the stem cells with a stem cell pretreatment substance (exendin-4) according to an embodiment of the present disclosure.
FIG. 6 is a graph illustrating an increase in the amount of exosomal RNA with the treatment of the stem cells with a stem cell pretreatment substance (exendin-4) according to an embodiment of the present disclosure.
FIG. 7 is a graph illustrating an increase of stem cells in proliferation with the treatment of the stem cells with stem cell pretreatment substance (phorbol 12-myristate 13-acetate (PMA)) according to an embodiment of the present disclosure.
FIGS. 8a and 8b are views illustrating an increase of stem cells in stemness with the treatment of the stem cells with stem cell pretreatment substance (phorbol 12-myristate 13-acetate (PMA)) according to an embodiment of the present disclosure.
FIG. 9 is a size distribution plot of exosomes after treatment with the stem cell pretreatment substance (phorbol 12-myristate 13-acetate (PMA)) according to an embodiment of the present disclosure.
FIG. 10 is a graph illustrating an increase in the number of exosomes with the treatment of the stem cells with a stem cell pretreatment substance (phorbol 12-myristate 13-acetate (PMA)) according to an embodiment of the present disclosure.
FIG. 11 is a graph illustrating an increase in the amount of exosomal proteins with the treatment of the stem cells with a stem cell pretreatment substance (phorbol 12-myristate 13-acetate (PMA)) according to an embodiment of the present disclosure.
FIG. 12 is a graph illustrating an increase in the amount of exosomal RNA with the treatment of the stem cells with a stem cell pretreatment substance (phorbol 12-myristate 13-acetate (PMA)) according to an embodiment of the present disclosure.
FIG. 13 is a graph illustrating an increase of stem cells in proliferation with the treatment of the stem cells with stem cell pretreatment substance (interferon-γ) according to an embodiment of the present disclosure.
FIGS. 14a and 14b are views illustrating an increase of stem cells in stemness with the treatment of the stem cells with stem cell pretreatment substance (interferon-γ) according to an embodiment of the present disclosure.
FIG. 15 is a size distribution plot of exosomes after treatment with the stem cell pretreatment substance (interferon-γ) according to an embodiment of the present disclosure.
FIG. 16 is a graph illustrating an increase in the number of exosomes with the treatment of the stem cells with a stem cell pretreatment substance (interferon-γ) according to an embodiment of the present disclosure.
FIG. 17 is a graph illustrating an increase in the amount of exosomal proteins with the treatment of the stem cells with a stem cell pretreatment substance (interferon-γ) according to an embodiment of the present disclosure.
FIG. 18 is a graph illustrating an increase in the amount of exosomal RNA with the treatment of the stem cells with a stem cell pretreatment substance (interferon-γ) according to an embodiment of the present disclosure.
FIG. 19 is a graph illustrating an increase of stem cells in proliferation with the treatment of the stem cells with stem cell pretreatment substance (tetrandrine) according to an embodiment of the present disclosure.
FIGS. 20a and 20b are views illustrating an increase of stem cells in stemness with the treatment of the stem cells with stem cell pretreatment substance (tetrandrine) according to an embodiment of the present disclosure.
FIG. 21 is a size distribution plot of exosomes after treatment with the stem cell pretreatment substance (tetrandrine) according to an embodiment of the present disclosure.
FIG. 22 is a graph illustrating an increase in the number of exosomes with the treatment of the stem cells with a stem cell pretreatment substance (tetrandrine) according to an embodiment of the present disclosure.
FIG. 23 is a graph illustrating an increase in the amount of exosomal proteins with the treatment of the stem cells with a stem cell pretreatment substance (tetrandrine) according to an embodiment of the present disclosure.
FIG. 24 is a graph illustrating an increase in the amount of exosomal RNA with the treatment of the stem cells with a stem cell pretreatment substance (tetrandrine) according to an embodiment of the present disclosure.
FIG. 25 is a graph illustrating an increase of stem cells in proliferation with the treatment of the stem cells with stem cell pretreatment substance (hyaluronic acid) according to an embodiment of the present disclosure.
FIG. 26a and 26b are views illustrating an increase of stem cells in stemness with the treatment of the stem cells with stem cell pretreatment substance (hyaluronic acid) according to an embodiment of the present disclosure.
FIG. 27 is a size distribution plot of exosomes after treatment with the stem cell pretreatment substance (hyaluronic acid) according to an embodiment of the present disclosure.
FIG. 28 is a graph illustrating an increase in the number of exosomes with the treatment of the stem cells with a stem cell pretreatment substance (hyaluronic acid) according to an embodiment of the present disclosure.
FIG. 29 is a graph illustrating an increase in the amount of exosomal proteins with the treatment of the stem cells with a stem cell pretreatment substance (hyaluronic acid) according to an embodiment of the present disclosure.
FIG. 30 is a graph illustrating an increase in the amount of exosomal RNA with the treatment of the stem cells with a stem cell pretreatment substance (hyaluronic acid) according to an embodiment of the present disclosure.
FIG. 31 is a graph illustrating an increase of stem cells in proliferation with the treatment of the stem cells with stem cell pretreatment substance (substance P) according to an embodiment of the present disclosure.
FIGS. 32a and 32b are views illustrating an increase of stem cells in stemness with the treatment of the stem cells with stem cell pretreatment substance (substance P) according to an embodiment of the present disclosure.
FIG. 33 is a size distribution plot of exosomes after treatment with the stem cell pretreatment substance (substance P) according to an embodiment of the present disclosure.
FIG. 34 is a graph illustrating an increase in the number of exosomes with the treatment of the stem cells with a stem cell pretreatment substance (substance P) according to an embodiment of the present disclosure.
FIG. 35 is a graph illustrating an increase in the amount of exosomal proteins with the treatment of the stem cells with a stem cell pretreatment substance (substance P) according to an embodiment of the present disclosure.
FIG. 36 is a graph illustrating an increase in the amount of exosomal RNA with the treatment of the stem cells with a stem cell pretreatment substance (substance P) according to an embodiment of the present disclosure.
FIG. 37 is a graph illustrating an increase of stem cells in proliferation with the treatment of the stem cells with stem cell pretreatment substance (resveratrol) according to an embodiment of the present disclosure.
FIGS. 38a and 38b are views illustrating an increase of stem cells in stemness with the treatment of the stem cells with stem cell pretreatment substance (resveratrol) according to an embodiment of the present disclosure.
FIG. 39 is a size distribution plot of exosomes after treatment with the stem cell pretreatment substance (resveratrol) according to an embodiment of the present disclosure.
FIG. 40 is a graph illustrating an increase in the number of exosomes with the treatment of the stem cells with a stem cell pretreatment substance (resveratrol) according to an embodiment of the present disclosure.
FIG. 41 is a graph illustrating an increase in the amount of exosomal proteins with the treatment of the stem cells with a stem cell pretreatment substance (resveratrol) according to an embodiment of the present disclosure.
FIG. 42 is a graph illustrating an increase in the amount of exosomal RNA with the treatment of the stem cells with a stem cell pretreatment substance (resveratrol) according to an embodiment of the present disclosure.
FIG. 43 is a graph illustrating an increase of stem cells in proliferation with the treatment of the stem cells with stem cell pretreatment substance (lanifibranor) according to an embodiment of the present disclosure.
FIGS. 44a and 44b are views illustrating an increase of stem cells in stemness with the treatment of the stem cells with stem cell pretreatment substance (lanifibranor) according to an embodiment of the present disclosure.
FIG. 45 is a size distribution plot of exosomes after treatment with the stem cell pretreatment substance (lanifibranor) according to an embodiment of the present disclosure.
FIG. 46 is a graph illustrating an increase in the number of exosomes with the treatment of the stem cells with a stem cell pretreatment substance (lanifibranor) according to an embodiment of the present disclosure.
FIG. 47 is a graph illustrating an increase in the amount of exosomal proteins with the treatment of the stem cells with a stem cell pretreatment substance (lanifibranor) according to an embodiment of the present disclosure.
FIG. 48 is a graph illustrating an increase in the amount of exosomal RNA with the treatment of the stem cells with a stem cell pretreatment substance (lanifibranor) according to an embodiment of the present disclosure.

### Best Mode for Carrying out the Invention

A composition for promoting production of stem cell-derived exosomes, the composition comprising at least one selected from the group consisting of exendin-4, phorbol 12-myristate 13-acetate (PMA), interferon-γ, tetrandrine, hyaluronic acid, substance P, resveratrol, and lanifibranor.

### Mode for Carrying out the Invention

A better understanding of the present disclosure will be obtained from the following Examples which are set forth to illustrate the present disclosure and are not to be construed as limiting the present disclosure.

### EXAMPLE 1. Isolation of Stem Cell-Derived Exosome According to Treatment with Exendin-4

### Exendin-4 pretreatment

In a culture medium [high glucose DMEM(Gibco, Cat no.11995-065); 10% fetal bovine serum (HyClone), 1% MEM non-essential amino acids solution (100X)(Gibco, Cat no.11140-050)] containing 20 nM of exendin-4, primary umbilical cord-derived mesenchymal stem cells (ATCC No. PCS-500-010; Lot No. 63822428) were cultured for one week.

### Additional culturing

After completion of culturing, the mesenchymal stem cells pretreated with exendin-4 were washed and additionally cultured for 72 hours in a culture medium supplemented with 10 % fetal bovine serum (FBS) free of exosomes. The reason of employing exosome-free FBS was to prevent FBS-derived exosomes other than those secreted from the cells from being incorporated because generally used FBS contained a large amount of exosomes derived from bovine serum.

### Exosome isolation

After 72 hours of culturing, the culture medium in which the pretreated mesenchymal stem cells were cultured was taken and centrifuged at 300xg for 10 min to remove residual cells and cell debris. The supernatant was filtered through a 0.22 µm filter, followed by centrifugation at 10,000xg and 4°C for 70 min in a high-speed centrifuge. The supernatant thus obtained was subjected again to ultracentrifugation at 100,000xg and 4°C for 90 min. The exosomes obtained as a pellet were diluted in PBS (phosphate buffered saline) before use in the following experiments.

### EXAMPLE 2. Isolation of Stem Cell-Derived Exosome According to Treatment with PMA

### PMA pretreatment

In a culture medium [high glucose DMEM (Gibco, Cat no.11995-065); 10% fetal bovine serum (HyClone), 1% MEM non-essential amino acids solution (100X) (Gibco, Cat no.11140-050)] containing 10 nM of PMA, primary umbilical cord-derived mesenchymal stem cells (ATCC No. PCS-500-010; Lot No. 63822428) were cultured for one week.

### Additional culturing

After completion of culturing, the mesenchymal stem cells pretreated with PMA were washed and additionally cultured for 72 hours in a culture medium supplemented with 10 % fetal bovine serum (FBS) free of exosomes. The reason of employing exosome-free FBS was to prevent FBS-derived exosomes other than those secreted from the cells from being incorporated because generally used FBS contained a large amount of exosomes derived from bovine serum.

### Exosome isolation

After 72 hours of culturing, the culture medium in which the pretreated mesenchymal stem cells were cultured was taken and centrifuged at 300xg for 10 min to remove residual cells and cell debris. The supernatant was filtered through a 0.22 µm filter, followed by centrifugation at 10,000xg and 4°C for 70 min in a high-speed centrifuge. The supernatant thus obtained was subjected again to ultracentrifugation at 100,000xg and 4°C for 90 min. The exosomes obtained as a pellet were diluted in PBS (phosphate buffered saline) before use in the following experiments.

### EXAMPLE 3. Isolation of Stem Cell-Derived Exosome According to Treatment with Interferon-γ

### Interferon-γ pretreatment

In a culture medium [high glucose DMEM (Gibco, Cat no.11995-065); 10% fetal bovine serum (HyClone), 1% MEM non-essential amino acids solution (100X) (Gibco, Cat no.11140-050)] containing 10 ng/mL interferon-γ, primary umbilical cord-derived mesenchymal stem cells (ATCC No. PCS-500-010; Lot No. 63822428) were cultured for one week.

### Additional culturing

After completion of culturing, the mesenchymal stem cells pretreated with interferon-γ were washed and additionally cultured for 72 hours in a culture medium supplemented with 10 % fetal bovine serum (FBS) free of exosomes. The reason of employing exosome-free FBS was to prevent FBS-derived exosomes other than those secreted from the cells from being incorporated because generally used FBS contained a large amount of exosomes derived from bovine serum.

### Exosome isolation

After 72 hours of culturing, the culture medium in which the pretreated mesenchymal stem cells were cultured was taken and centrifuged at 300xg for 10 min to remove residual cells and cell debris. The supernatant was filtered through a 0.22 µm filter, followed by centrifugation at 10,000xg and 4°C for 70 min in a high-speed centrifuge. The supernatant thus obtained was subjected again to ultracentrifugation at 100,000xg and 4°C for 90 min. The exosomes obtained as a pellet were diluted in PBS (phosphate buffered saline) before use in the following experiments.

### EXAMPLE 4. Isolation of Stem Cell-Derived Exosome According to Treatment with Tetrandrine

### Tetrandrine pretreatment

In a culture medium [high glucose DMEM (Gibco, Cat no.11995-065); 10% fetal bovine serum (HyClone), 1% MEM non-essential amino acids solution (100X) (Gibco, Cat no.11140-050)] containing 10 µM of tetrandrine, primary umbilical cord-derived mesenchymal stem cells (ATCC No. PCS-500-010; Lot No. 63822428) were cultured for one week.

### Additional culturing

After completion of culturing, the mesenchymal stem cells pretreated with tetrandrine were washed and additionally cultured for 72 hours in a culture medium supplemented with 10 % fetal bovine serum (FBS) free of exosomes. The reason of employing exosome-free FBS was to prevent FBS-derived exosomes other than those secreted from the cells from being incorporated because generally used FBS contained a large amount of exosomes derived from bovine serum.

### Exosome isolation

After 72 hours of culturing, the culture medium in which the pretreated mesenchymal stem cells were cultured was taken and centrifuged at 300xg for 10 min to remove residual cells and cell debris. The supernatant was filtered through a 0.22 µm filter, followed by centrifugation at 10,000xg and 4°C for 70 min in a high-speed centrifuge. The supernatant thus obtained was subjected again to ultracentrifugation at 100,000xg and 4°C for 90 min. The exosomes obtained as a pellet were diluted in PBS (phosphate buffered saline) before use in the following experiments.

### EXAMPLE 5. Isolation of Stem Cell-Derived Exosome According to Treatment with Hyaluronic Acid

### Hyaluronic acid pretreatment

In a culture medium [high glucose DMEM (Gibco, Cat no.11995-065); 10% fetal bovine serum (HyClone), 1% MEM non-essential amino acids solution (100X) (Gibco, Cat no.11140-050)] containing 10 µg/mL hyaluronic acid, primary umbilical cord-derived mesenchymal stem cells (ATCC No. PCS-500-010; Lot No. 63822428) were cultured for one week.

### Additional culturing

After completion of culturing, the mesenchymal stem cells pretreated with hyaluronic acid were washed and additionally cultured for 72 hours in a culture medium supplemented with 10 % fetal bovine serum (FBS) free of exosomes. The reason of employing exosome-free FBS was to prevent FBS-derived exosomes other than those secreted from the cells from being incorporated because generally used FBS contained a large amount of exosomes derived from bovine serum.

### Exosome isolation

After 72 hours of culturing, the culture medium in which the pretreated mesenchymal stem cells were cultured was taken and centrifuged at 300xg for 10 min to remove residual cells and cell debris. The supernatant was filtered through a 0.22 µm filter, followed by centrifugation at 10,000xg and 4°C for 70 min in a high-speed centrifuge. The supernatant thus obtained was subjected again to ultracentrifugation at 100,000xg and 4°C for 90 min. The exosomes obtained as a pellet were diluted in PBS (phosphate buffered saline) before use in the following experiments.

### EXAMPLE 6. Isolation of Stem Cell-Derived Exosome According to Treatment with Substance P

### Substance P pretreatment

In a culture medium [high glucose DMEM (Gibco, Cat no.11995-065); 10% fetal bovine serum (HyClone), 1% MEM non-essential amino acids solution (100X) (Gibco, Cat no.11140-050)] containing 30 nM of substance P, primary umbilical cord-derived mesenchymal stem cells (ATCC No. PCS-500-010; Lot No. 63822428) were cultured for one week.

### Additional culturing

After completion of culturing, the mesenchymal stem cells pretreated with substance P were washed and additionally cultured for 72 hours in a culture medium supplemented with 10 % fetal bovine serum (FBS) free of exosomes. The reason of employing exosome-free FBS was to prevent FBS-derived exosomes other than those secreted from the cells from being incorporated because generally used FBS contained a large amount of exosomes derived from bovine serum.

### Exosome isolation

After 72 hours of culturing, the culture medium in which the pretreated mesenchymal stem cells were cultured was taken and centrifuged at 300xg for 10 min to remove residual cells and cell debris. The supernatant was filtered through a 0.22 µm filter, followed by centrifugation at 10,000xg and 4°C for 70 min in a high-speed centrifuge. The supernatant thus obtained was subjected again to ultracentrifugation at 100,000xg and 4°C for 90 min. The exosomes obtained as a pellet were diluted in PBS (phosphate buffered saline) before use in the following experiments.

### EXAMPLE 7. Isolation of Stem Cell-Derived Exosome According to Treatment with Resveratrol

### Resveratrol pretreatment

In a culture medium [high glucose DMEM (Gibco, Cat no.11995-065); 10% fetal bovine serum (HyClone), 1% MEM non-essential amino acids solution (100X) (Gibco, Cat no.11140-050)] containing 10 nM of resveratrol, primary umbilical cord-derived mesenchymal stem cells (ATCC No. PCS-500-010; Lot No. 63822428) were cultured for one week.

### Additional culturing

After completion of culturing, the mesenchymal stem cells pretreated with resveratrol were washed and additionally cultured for 72 hours in a culture medium supplemented with 10 % fetal bovine serum (FBS) free of exosomes. The reason of employing exosome-free FBS was to prevent FBS-derived exosomes other than those secreted from the cells from being incorporated because generally used FBS contained a large amount of exosomes derived from bovine serum.

### Exosome isolation

After 72 hours of culturing, the culture medium in which the pretreated mesenchymal stem cells were cultured was taken and centrifuged at 300xg for 10 min to remove residual cells and cell debris. The supernatant was filtered through a 0.22 µm filter, followed by centrifugation at 10,000xg and 4°C for 70 min in a high-speed centrifuge. The supernatant thus obtained was subjected again to ultracentrifugation at 100,000xg and 4°C for 90 min. The exosomes obtained as a pellet were diluted in PBS (phosphate buffered saline) before use in the following experiments.

### EXAMPLE 8. Stem Cell-Derived Exosome According to Treatment with Lanifibranor

### Lanifibranor pretreatment

In a culture medium [high glucose DMEM (Gibco, Cat no.11995-065); 10% fetal bovine serum (HyClone), 1% MEM non-essential amino acids solution (100X) (Gibco, Cat no.11140-050)] containing 10 µM of lanifibranor, primary umbilical cord-derived mesenchymal stem cells (ATCC No. PCS-500-010; Lot No. 63822428) were cultured for one week.

### Additional culturing

After completion of culturing, the mesenchymal stem cells pretreated with lanifibranor were washed and additionally cultured for 72 hours in a culture medium supplemented with 10 % fetal bovine serum (FBS) free of exosomes. The reason of employing exosome-free FBS was to prevent FBS-derived exosomes other than those secreted from the cells from being incorporated because generally used FBS contained a large amount of exosomes derived from bovine serum.

### Exosome isolation

After 72 hours of culturing, the culture medium in which the pretreated mesenchymal stem cells were cultured was taken and centrifuged at 300xg for 10 min to remove residual cells and cell debris. The supernatant was filtered through a 0.22 µm filter, followed by centrifugation at 10,000xg and 4°C for 70 min in a high-speed centrifuge. The supernatant thus obtained was subjected again to ultracentrifugation at 100,000xg and 4°C for 90 min. The exosomes obtained as a pellet were diluted in PBS (phosphate buffered saline) before use in the following experiments.

### EXPERIMENTAL EXAMPLE 1. Functional Reinforcement of Stem Cells by Treatment with Exendin-4

### 1-1. Increase of stem cell proliferation by treatment with exendin-4

Mesenchymal stem cells and the exendin-4-pretreated mesenchymal stem cells of the Example were each seeded at a density of 3,000 cells/well into 96-well plates containing 100 µL of a culture medium [high glucose DMEM (Gibco, Cat no.11995-065), 10% fetal bovine serum (HyClone), 1% MEM non-essential amino acids solution (100X) (Gibco, Cat no.11140-050)] per well and cultured for 24 hours in a CO₂ incubator. Then, CCK solution (10 µL/well) was added, and incubation was continued for 4 hours in the CO₂ incubator, followed by reading absorbance at a wavelength of 450 nm (420-480 nm) to determine proliferation.

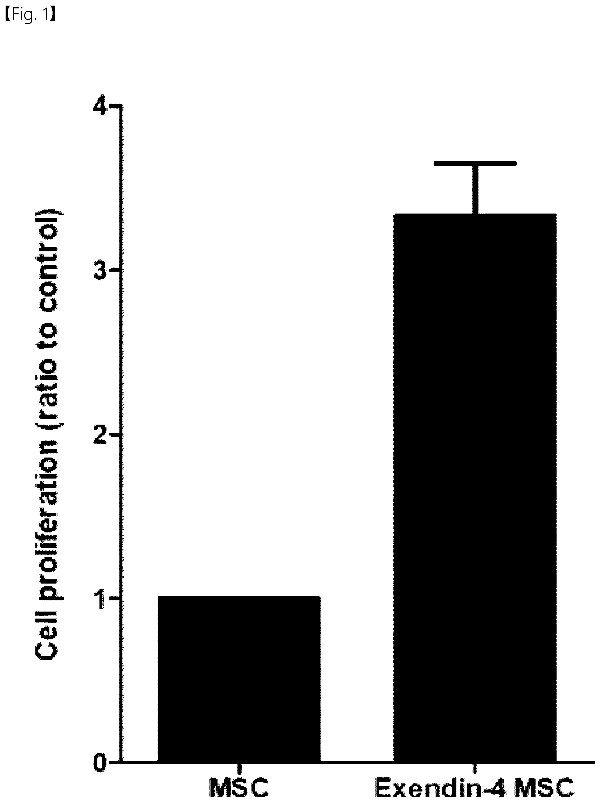

As can be seen in FIG. 1, the mesenchymal stem cells pretreated with the stem cell pretreatment substance [exendin-4] increased in proliferation by about 340 %, with the ratio from 1 to 3.4 relative to the non-treated mesenchymal stem cells.

### 1-2. Increase of stem cell stemness by treatment with exendin-4

Mesenchymal stem cells and the exendin-4-pretreated mesenchymal stem cells of the Example were each seeded at a density of 1,000 cells/dish into 100-mm cell culture dishes containing 100 mL of a culture medium [high glucose DMEM (Gibco, Cat no.11995-065), 10% fetal bovine serum (HyClone), 1% MEM non-essential amino acids solution (100X) (Gibco, Cat no.11140-050)] per dish and cultured for 21 days. The cell-attached dishes were washed twice with PBS, followed by fixation with 95% methanol at room temperature for about 2 min. The fixed cells were washed three times with PBS and stained with 0.5% crystal violet [(sigma, C-3886, USA) 5g, methanol 100 ml] for 5 min. After water washing and drying at room temperature, colonies, each composed of 50 or more cells, were counted for comparison.

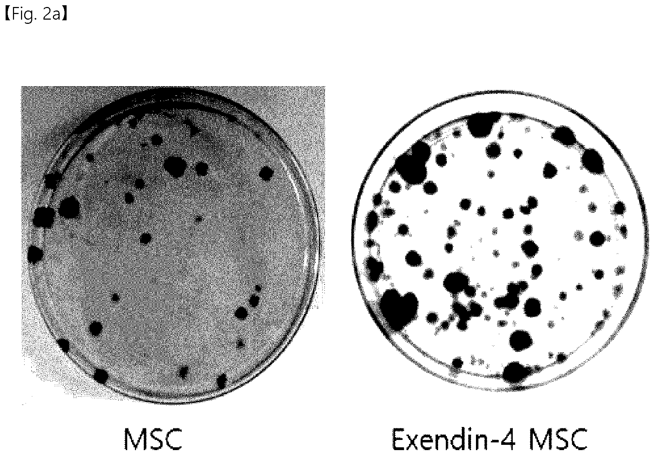

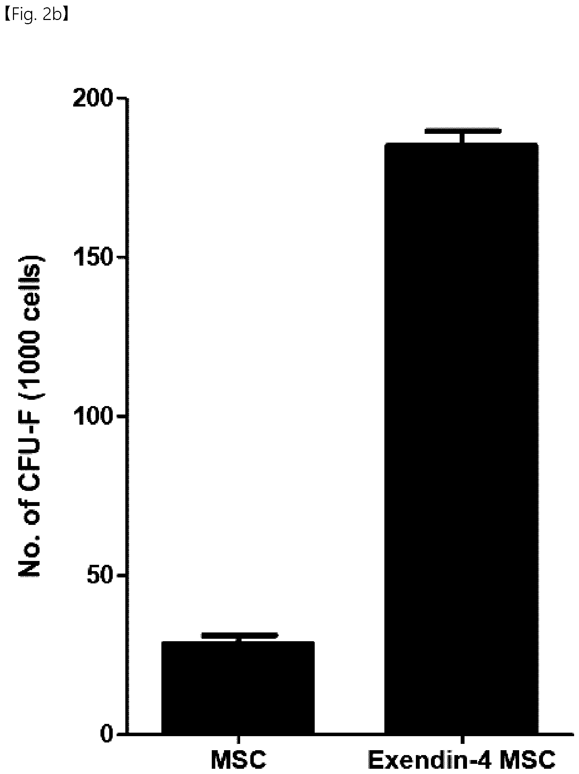

As can be seen in FIGS. 2a and 2b, more colonies were formed by the mesenchymal stem cells pretreated with the stem cell pretreatment substance [exendin-4] (185 colonies) than the non-treated mesenchymal stem cells (28.6), with CFU-F increasing by about 647%.

### EXPERIMENTAL EXAMPLE 2. Increase of Exosome Productivity by Treatment with Exendin-4

### 2-1. Increase in number of exosomes by treatment with exendin-4

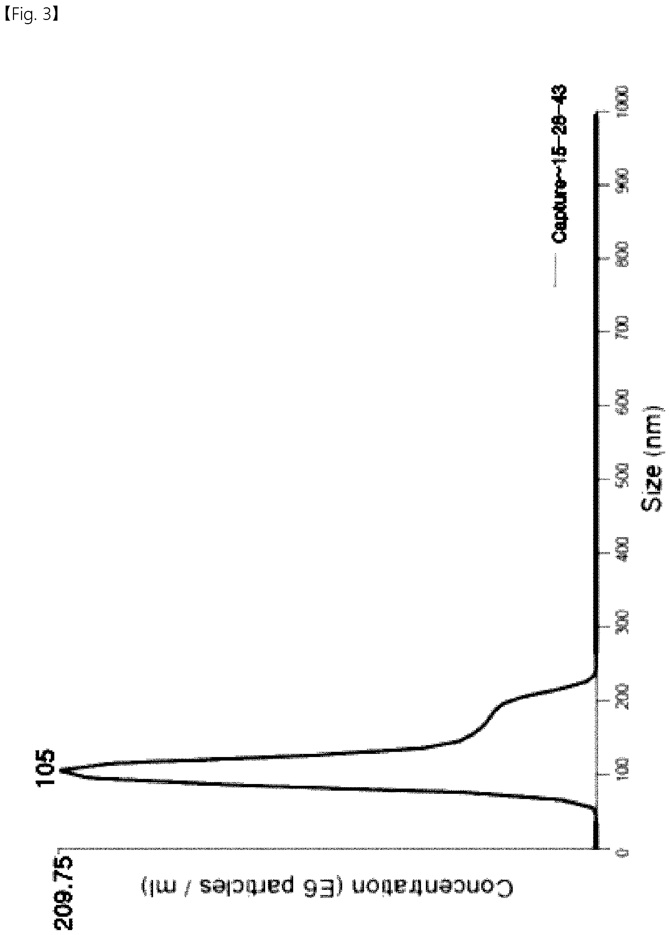

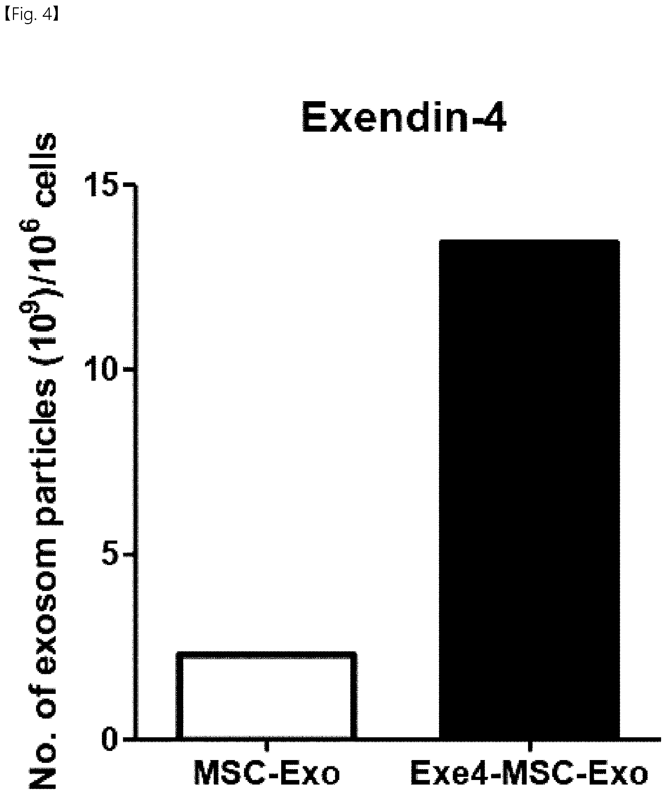

The exosomes isolated in the Example were counted through the nanoparticle tracking assay (NanoSight NS300, Malvern). For comparison, exosomes were counted in the same manner with the exception that the cells were not pretreated with any pretreatment substance. The results are given in Table 1 and FIGS. 3 and 4.

**TABLE 1**

| Yield (per 10⁶ cells) | MSC-Exo | Exe4-MSC-Exo |
|---|---|---|
| No. of exosome particles (10⁹) | 2.30 | 13.45 |

| | | |
|---|---|---|
| (MSC: non-treated mesenchymal stem cells, Exe4-MSC: Exendin-4-treated mesenchymal stem cells) | | |

As can be seen in Table 1 and FIG. 4, the stem cell pretreatment substance [exendin-4]-treated mesenchymal stem cells produced about 5.85-fold more exosomes than the non-treated mesenchymal stem cells.

From the results, it is understood that treatment of mesenchymal stem cells with the stem cell pretreatment substance according to the present disclosure increases the quantity of exosomes produced by the mesenchymal stem cells.

### 2-2. Increase in content of exosomal protein by treatment with exendin-4

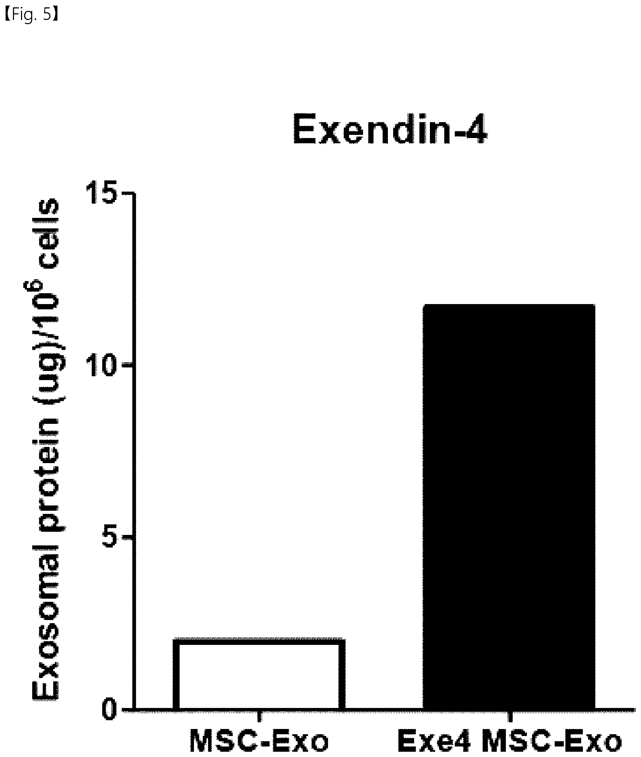

Proteins were isolated from the exosomes isolated in the Examples, using an exosome protein isolation kit (total exosome RNA and protein isolation kit, Invitrogen), and quantitatively measured by reading absorbance at 595 nm through Bradford analysis. For comparison, proteins were isolated from exosomes and quantitated in the same manner with the exception that the cells were not pretreated with any pretreatment substance. The results are given in Table 2 and FIG. 5.

**TABLE 2**

| Yield (per 10⁶ cells) | MSC-Exo | Exe4-MSC-Exo |
|---|---|---|
| Exosomal protein(pg) | 2.00 | 10.69 |

| | | |
|---|---|---|
| (MSC: non-treated mesenchymal stem cells, Exe4-MSC: Exendin-4-treated mesenchymal stem cells) | | |

As can be seen in Table 2 and FIG. 5, the stem cell pretreatment substance [exendin-4]-treated mesenchymal stem cells produced about 5.4-fold greater exosomal proteins than the non-treated mesenchymal stem cells.

From the results, it is understood that the content of exosomal proteins as well as the number of exosomes are increased by treatment of mesenchymal stem cells with the stem cell pretreatment substance according to the present disclosure.

### 2-3. Increase in content of exosomal RNA by treatment with exendin-4

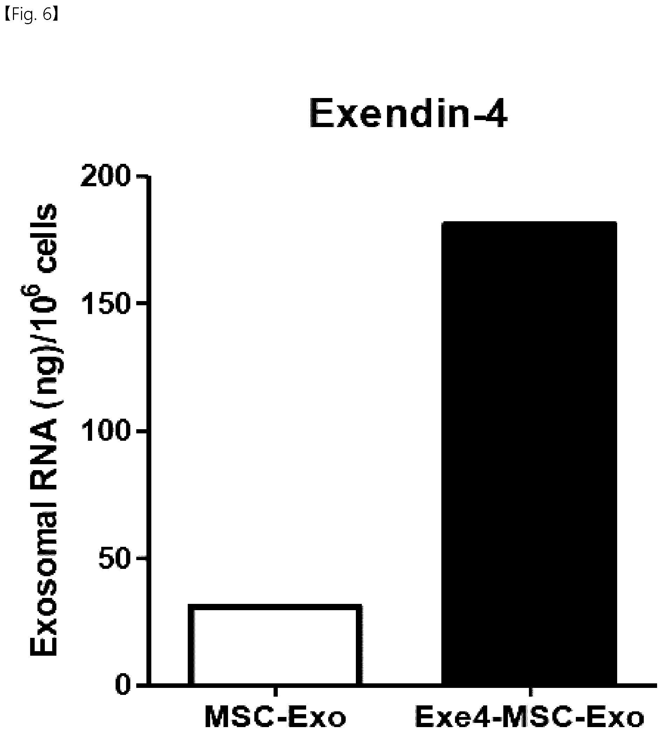

Using an RNA isolation kit (total exosome RNA and protein isolation kit, Invitrogen), total exosomal RNA was isolated from the exosomes isolated in the Example, followed by measuring RNA concentrations with the aid of a NanoDrop spectrometer. For comparison, total exosomal RNA was isolated from exosomes and quantitated in the same manner with the exception that the cells were not pretreated with any pretreatment substance. The results are given in Table 3 and FIG. 6.

**TABLE 3**

| Yield (per 10⁶ cells) | MSC-Exo | Exe4-MSC-Exo |
|---|---|---|
| Exosomal RNA (ng) | 31.00 | 174.34 |

| | | |
|---|---|---|
| (MSC: non-treated mesenchymal stem cells, Exe4-MSC: Exendin-4-treated mesenchymal stem cells) | | |

As can be seen in Table 3 and FIG. 6, the stem cell pretreatment substance [exendin-4]-treated mesenchymal stem cells produced about 5.6-fold greater exosomal RNA than the non-treated mesenchymal stem cells.

From the results, it is understood that the content of exosomal RNA as well as the number of exosomes are increased by treatment of mesenchymal stem cells with the stem cell pretreatment substance according to the present disclosure.

### EXPERIMENTAL EXAMPLE 3. Functional Reinforcement of Stem Cells by Treatment with PMA

### 3-1. Increase of stem cell proliferation by treatment with PMA

Mesenchymal stem cells and the PMA-pretreated mesenchymal stem cells of the Example were each seeded at a density of 3,000 cells/well into 96-well plates containing 100 µL of a culture medium [high glucose DMEM (Gibco, Cat no.11995-065), 10% fetal bovine serum (HyClone), 1% MEM non-essential amino acids solution (100X) (Gibco, Cat no.11140-050)] per well and cultured for 24 hours in a CO₂ incubator. Then, CCK solution (10 µL/well) was added, and incubation was continued for 4 hours in the CO₂ incubator, followed by reading absorbance at a wavelength of 450 nm (420-480 nm) to determine proliferation.

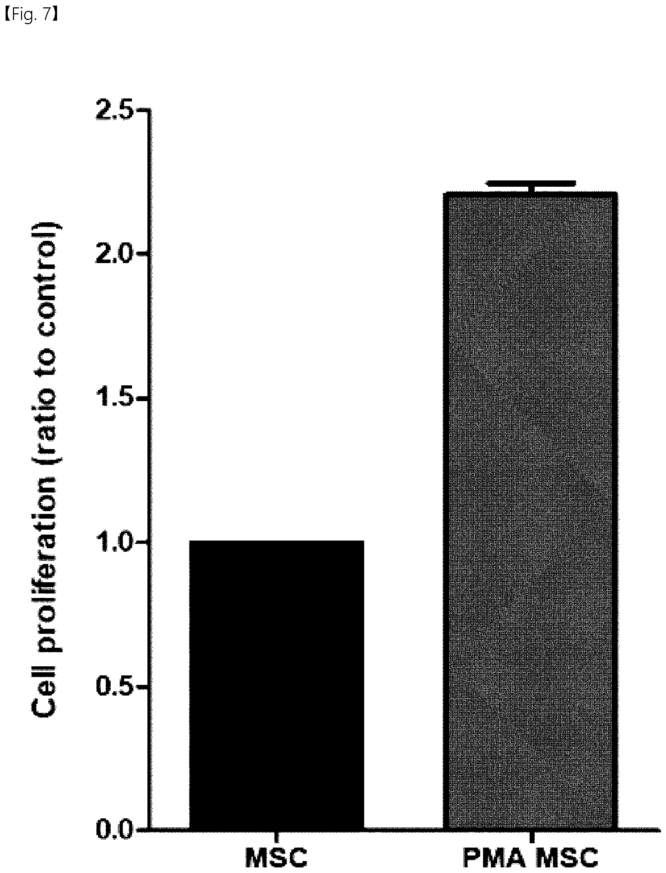

As can be seen in FIG. 7, the mesenchymal stem cells pretreated with the stem cell pretreatment substance [PMA] increased in proliferation by about 220 %, with the ratio from 1 to 2.2 relative to the non-treated mesenchymal stem cells.

### 3-2. Increase of stem cell sternness by treatment with PMA

Mesenchymal stem cells and the PMA-pretreated mesenchymal stem cells of the Example were each seeded at a density of 1,000 cells/dish into 100-mm cell culture dishes containing 100 mL of a culture medium [high glucose DMEM (Gibco, Cat no.11995-065), 10% fetal bovine serum (HyClone), 1% MEM non-essential amino acids solution (100X) (Gibco, Cat no.11140-050)] per dish and cultured for 21 days. The cell-attached dishes were washed twice with PBS, followed by fixation with 95% methanol at room temperature for about 2 min. The fixed cells were washed three times with PBS and stained with 0.5% crystal violet [(sigma, C-3886, USA) 5g, methanol 100 ml] for 5 min. After water washing and drying at room temperature, colonies, each composed of 50 or more cells, were counted for comparison.

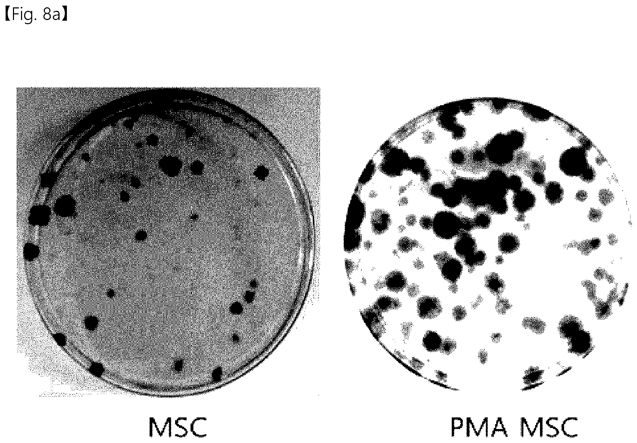

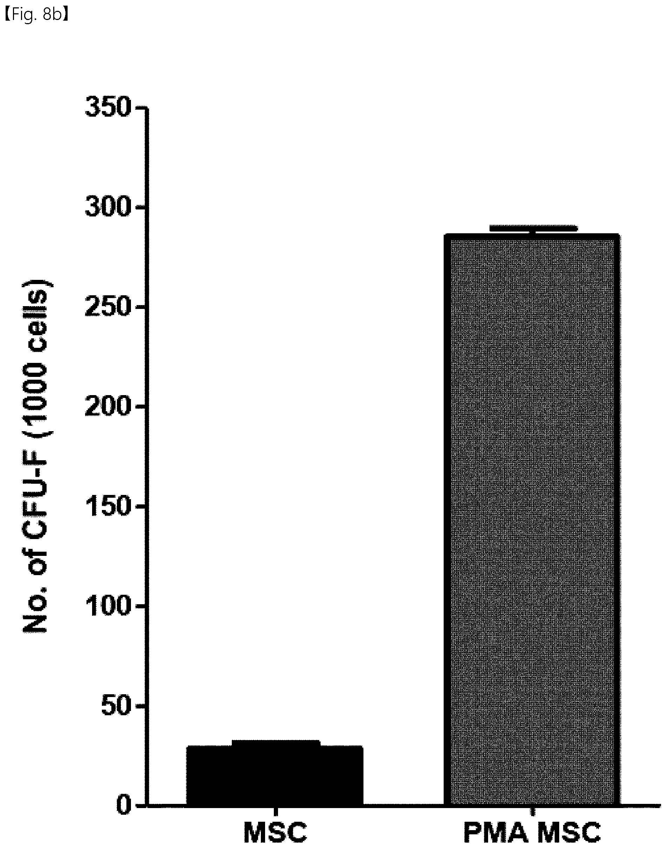

As can be seen in FIGS. 8a and 8b, more colonies were formed by the mesenchymal stem cells pretreated with the stem cell pretreatment substance [PMA] (285.7 colonies) than the non-treated mesenchymal stem cells (28.6), with CFU-F increasing by about 996.5%.

### EXPERIMENTAL EXAMPLE 4. Increase of Exosome Productivity by Treatment with PMA

### 4-1. Increase in number of exosomes by treatment with PMA

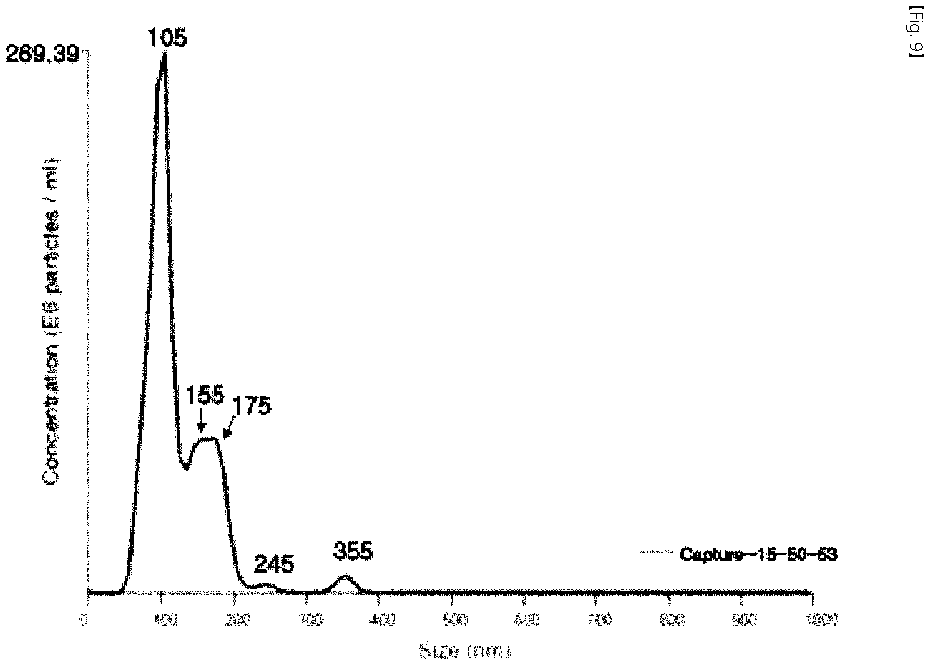

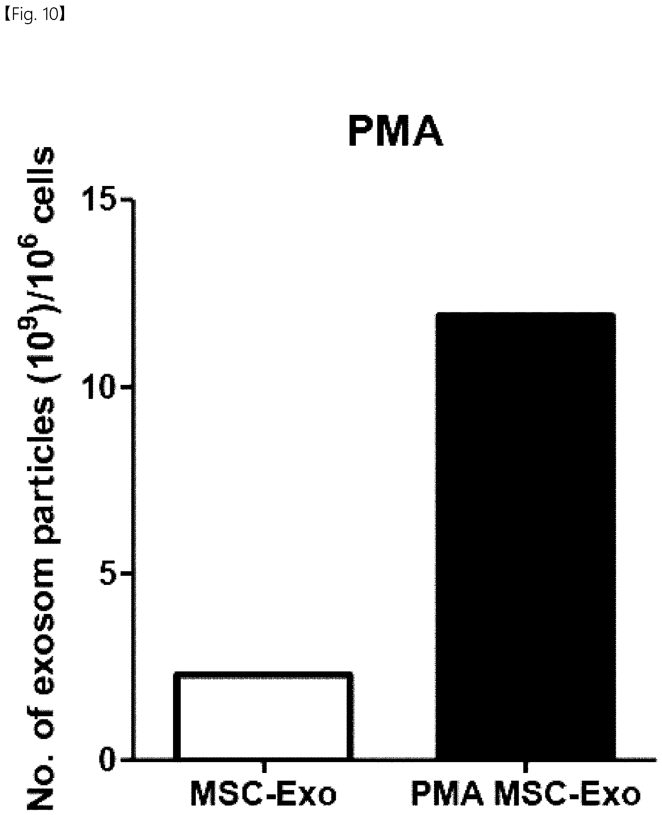

The exosomes isolated in the Example were counted the nanoparticle tracking assay (NanoSight NS300, Malvern). For comparison, exosomes were counted in the same manner with the exception that the cells were not pretreated with any pretreatment substance. The results are given in Table 4 and FIGS. 9 and 10.

**TABLE 4**

| Yield (per 10⁶ cells) | MSC-Exo | PMA MSC-Exo |
|---|---|---|
| No. of exosome particles (10⁹) | 2.3 | 11.90 |

| | | |
|---|---|---|
| (MSC: non-treated mesenchymal stem cells, PMA MSC: PMA-treated mesenchymal stem cells) | | |

As can be seen in Table 4 and FIG. 10, the stem cell pretreatment substance [PMA]-treated mesenchymal stem cells produced about 5.2-fold more exosomes than the non-treated mesenchymal stem cells.

From the results, it is understood that treatment of mesenchymal stem cells with the stem cell pretreatment substance according to the present disclosure increases the quantity of exosomes produced by the mesenchymal stem cells.

### 4-2. Increase in content of exosomal protein by treatment with PMA

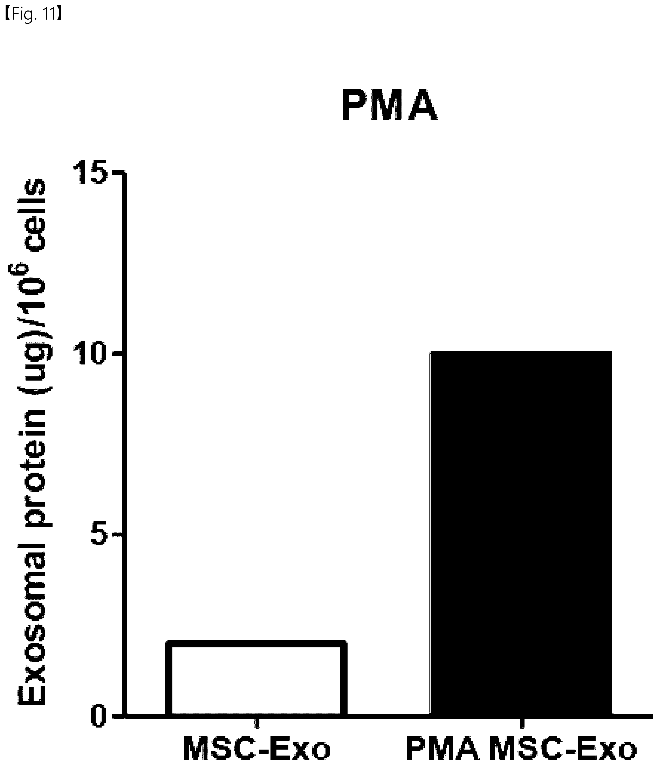

Proteins were isolated from the exosomes isolated in the Examples, using an exosome protein isolation kit (total exosome RNA and protein isolation kit, Invitrogen), and quantitatively measured by reading absorbance at 595 nm through Bradford analysis. For comparison, proteins were isolated from exosomes and quantitated in the same manner with the exception that the cells were not pretreated with any pretreatment substance. The results are given in Table 5 and FIG. 11.

**TABLE 5**

| Yield (per 10⁶ cells) | MSC-Exo | PMA MSC-Exo |
|---|---|---|
| Exosomal protein (ug) | 2.0 | 10.0 |

| | | |
|---|---|---|
| (MSC: non-treated mesenchymal stem cells, PMA: PMA-treated mesenchymal stem cells) | | |

As can be seen in Table 5 and FIG. 11, the stem cell pretreatment substance [PMA]-treated mesenchymal stem cells produced about 5-fold greater exosomal proteins than the non-treated mesenchymal stem cells.

From the results, it is understood that the content of exosomal proteins as well as the number of exosomes are increased by treatment of mesenchymal stem cells with the stem cell pretreatment substance according to the present disclosure.

### 4-3. Increase in content of exosomal RNA by treatment with PMA

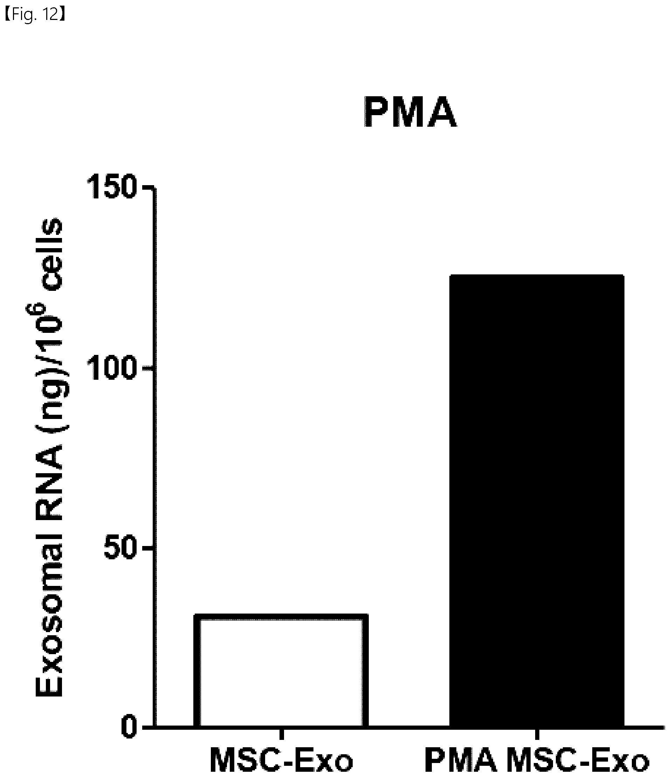

Using an RNA isolation kit (total exosome RNA and protein isolation kit, Invitrogen), total exosomal RNA was isolated from the exosomes isolated in the Example, followed by measuring RNA concentrations with the aid of a NanoDrop spectrometer. For comparison, total exosomal RNA was isolated from exosomes and quantitated in the same manner with the exception that the cells were not pretreated with any pretreatment substance. The results are given in Table 6 and FIG. 12.

**TABLE 6**

| Yield (per 10⁶ cells) | MSC-Exo | PMA MSC-Exo |
|---|---|---|
| Exosomal RNA (ng) | 31.0 | 125.0 |

| | | |
|---|---|---|
| (MSC: non-treated mesenchymal stem cells, PMA-MSC: PMA-treated mesenchymal stem cells) | | |

As can be seen in Table 6 and FIG. 12, the stem cell pretreatment substance [PMA]-treated mesenchymal stem cells produced about 4-fold greater exosomal RNA than the non-treated mesenchymal stem cells.

From the results, it is understood that the content of exosomal RNA as well as the number of exosomes are increased by treatment of mesenchymal stem cells with the stem cell pretreatment substance according to the present disclosure.

### EXPERIMENT EXAMPLE 5. Functional Reinforcement of Stem Cells by Treatment with Interferon-γ

### 5-1. Increase of stem cell proliferation by treatment with interferon-γ

Mesenchymal stem cells and the interferon-γ-pretreated mesenchymal stem cells of the Example were each seeded at a density of 3,000 cells/well into 96-well plates containing 100 µL of a culture medium [high glucose DMEM (Gibco, Cat no.11995-065), 10% fetal bovine serum (HyClone), 1% MEM non-essential amino acids solution (100X) (Gibco, Cat no.11140-050)] per well and cultured for 24 hours in a CO₂ incubator. Then, CCK solution (10 µL/well) was added, and incubation was continued for 4 hours in the CO₂ incubator, followed by reading absorbance at a wavelength of 450 nm (420-480 nm) to determine proliferation.

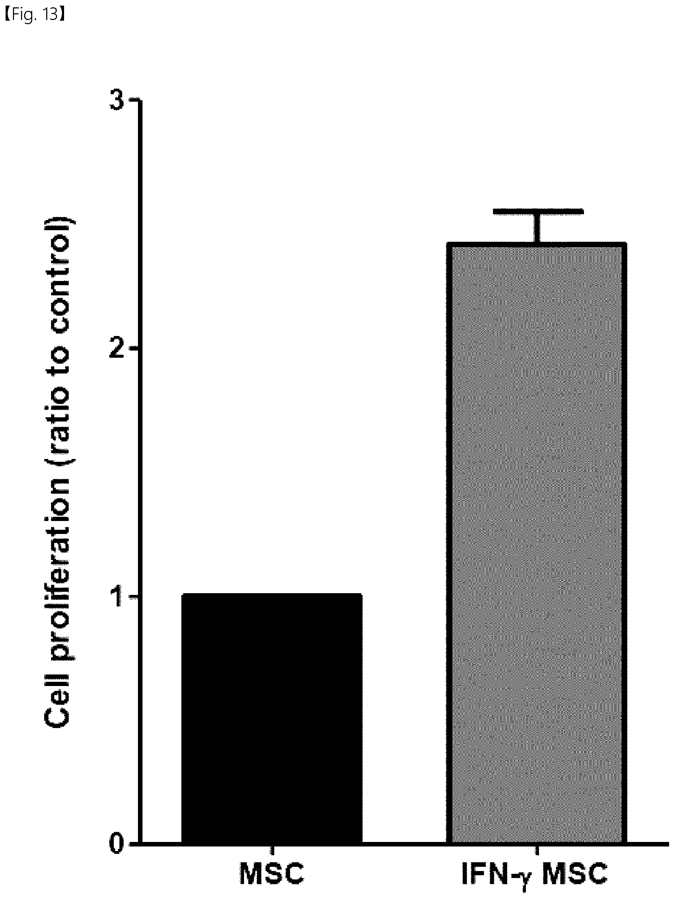

As can be seen in FIG. 13, the mesenchymal stem cells pretreated with the stem cell pretreatment substance [IFNγ] increased in proliferation by about 242 %, with the ratio from 1 to 2.42 relative to the non-treated mesenchymal stem cells.

### 5-2. Increase of stem cell sternness by treatment with interferon-γ

Mesenchymal stem cells and the interferon-γ-pretreated mesenchymal stem cells of the Example were each seeded at a density of 1,000 cells/dish into 100-mm cell culture dishes containing 100 mL of a culture medium [high glucose DMEM (Gibco, Cat no.11995-065), 10% fetal bovine serum (HyClone), 1% MEM non-essential amino acids solution (100X) (Gibco, Cat no.11140-050)] per dish and cultured for 21 days. The cell-attached dishes were washed twice with PBS, followed by fixation with 95% methanol at room temperature for about 2 min. The fixed cells were washed three times with PBS and stained with 0.5% crystal violet [(sigma, C-3886, USA) 5g, methanol 100 ml] for 5 min. After water washing and drying at room temperature, colonies, each composed of 50 or more cells, were counted for comparison.

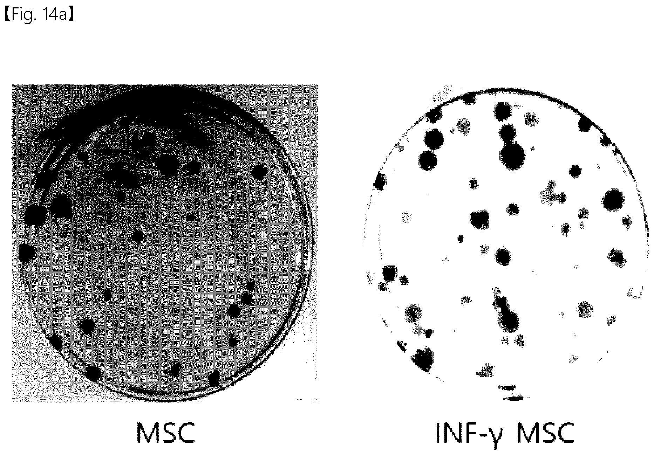

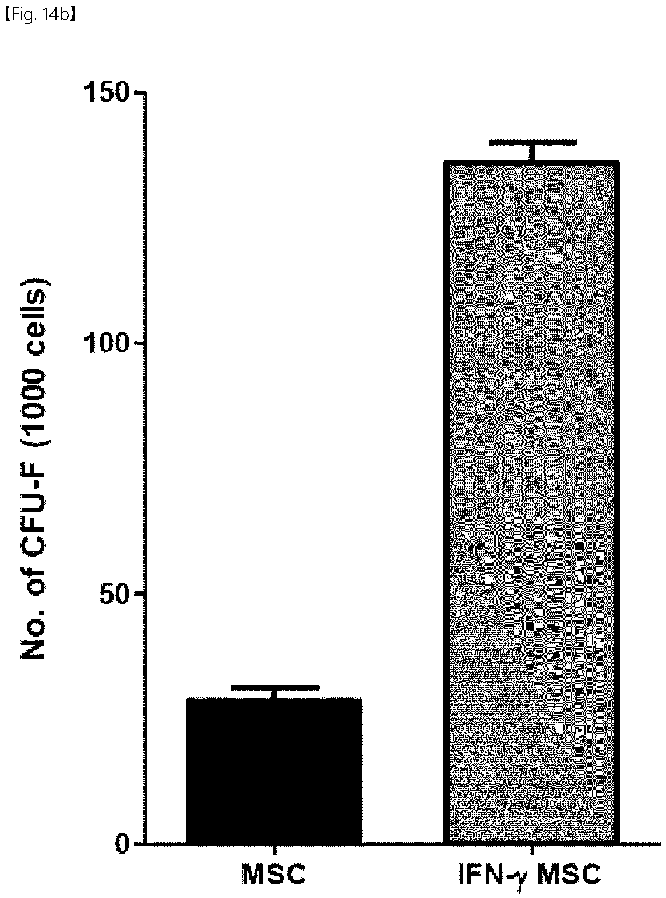

As can be seen in FIGS. 14a and 14b, more colonies were formed by the mesenchymal stem cells pretreated with the stem cell pretreatment substance [IFNγ] (136 colonies) than the non-treated mesenchymal stem cells (28.6), with CFU-F increasing by about 476%.

### EXPERIMENTAL EXAMPLE 6. Increase of Exosome Productivity by Treatment with Interferon-γ

### 6-1. Increase in number of exosomes by treatment with interferon-γ

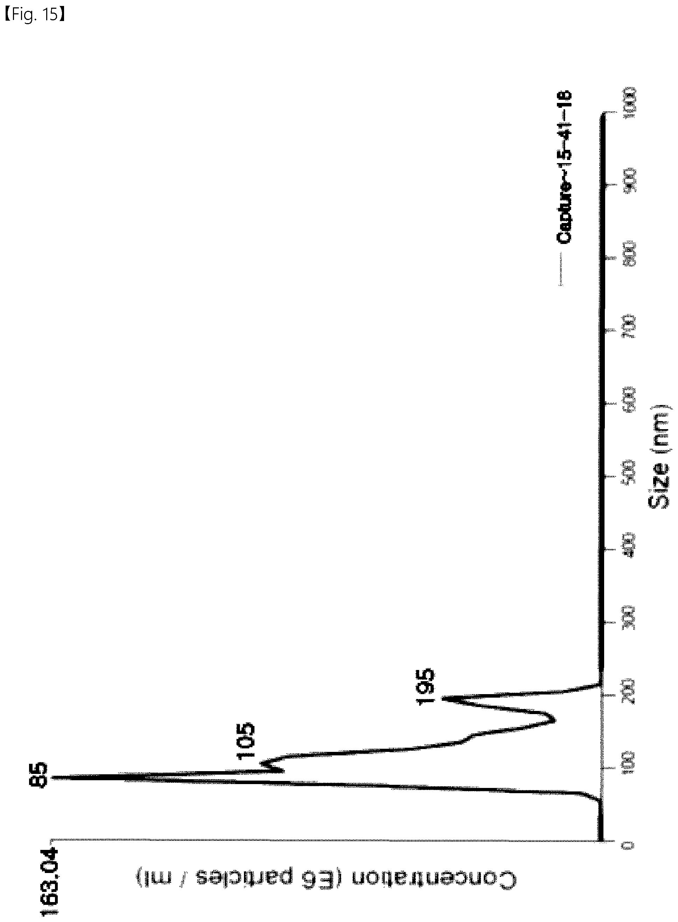

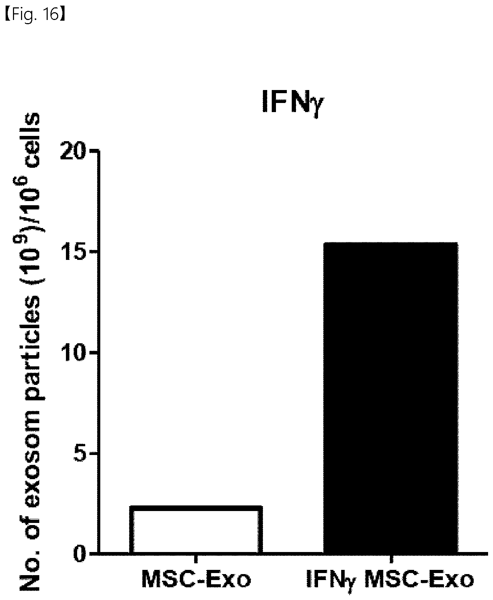

The exosomes isolated in the Example were counted the nanoparticle tracking assay (NanoSight NS300, Malvern). For comparison, exosomes were counted in the same manner with the exception that the cells were not pretreated with any pretreatment substance. The results are given in Table 7 and FIGS. 15 and 16.

**TABLE 7**

| Yield(per 10⁶ Cells) | MSC-Exo | IFNγ MSC-Exo |
|---|---|---|
| No. of exosome particles (10⁹) | 2.3 | 15.4 |

| | | |
|---|---|---|
| (MSC: non-treated mesenchymal stem cells, IFNγ MSC: IFNγ-treated mesenchymal stem cells) | | |

As can be seen in Table 7 and FIG. 16, the stem cell pretreatment substance [interferon-γ]-treated mesenchymal stem cells produced about 6.7-fold more exosomes than the non-treated mesenchymal stem cells.

From the results, it is understood that treatment of mesenchymal stem cells with the stem cell pretreatment substance according to the present disclosure increases the quantity of exosomes produced by the mesenchymal stem cells.

### 6-2. Increase in content of exosomal protein by treatment with interferon-γ

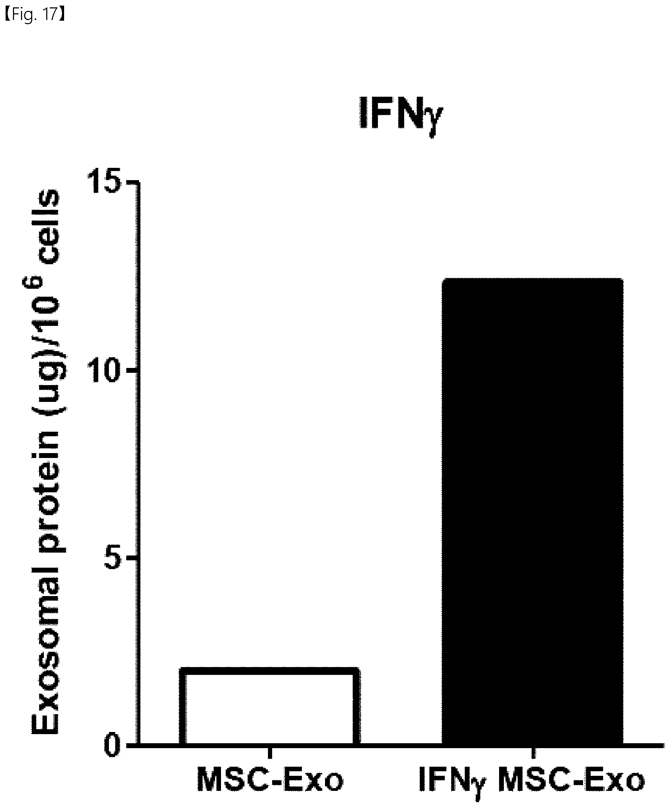

Proteins were isolated from the exosomes isolated in the Examples, using an exosome protein isolation kit (total exosome RNA and protein isolation kit, Invitrogen), and quantitatively measured by reading absorbance at 595 nm through Bradford analysis. For comparison, proteins were isolated from exosomes and quantitated in the same manner with the exception that the cells were not pretreated with any pretreatment substance. The results are given in Table 8 and FIG. 17.

**TABLE 8**

| Yield (per 10⁶ cells) | MSC-Exo | IFNγ MSC-Exo |
|---|---|---|
| Exosomal protein (ug) | 2.0 | 12.4 |

| | | |
|---|---|---|
| (MSC: non-treated mesenchymal stem cells, IFNγ MSC: IFNγ-treated mesenchymal stem cells) | | |

As can be seen in Table 8 and FIG. 17, the stem cell pretreatment substance [interferon-γ]-treated mesenchymal stem cells produced about 6.2-fold greater exosomal proteins than the non-treated mesenchymal stem cells.

From the results, it is understood that the content of exosomal proteins as well as the number of exosomes are increased by treatment of mesenchymal stem cells with the stem cell pretreatment substance according to the present disclosure.

### 6-3. Increase in content of exosomal RNA by treatment with interferon-γ

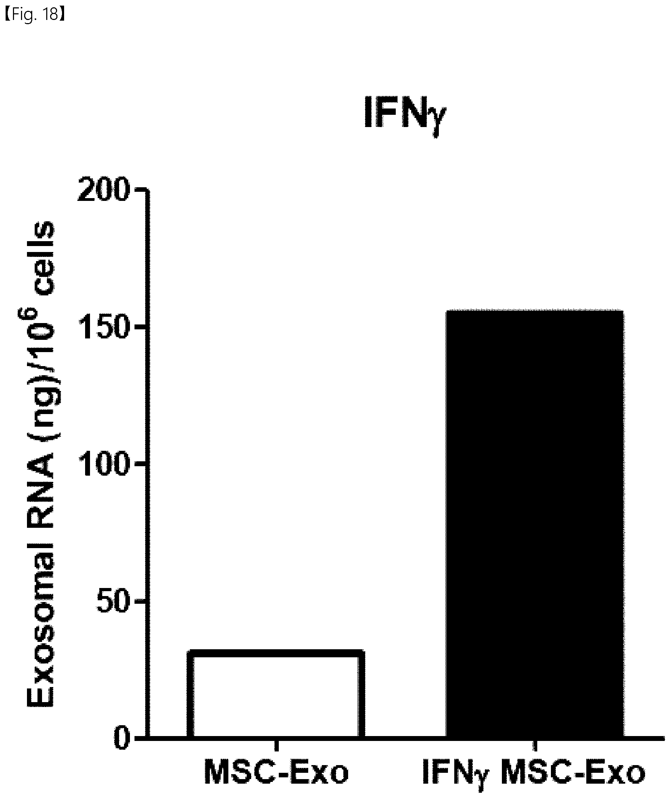

Using an RNA isolation kit (total exosome RNA and protein isolation kit, Invitrogen), total exosomal RNA was isolated from the exosomes isolated in the Example, followed by measuring RNA concentrations with the aid of a NanoDrop spectrometer. For comparison, total exosomal RNA was isolated from exosomes and quantitated in the same manner with the exception that the cells were not pretreated with any pretreatment substance. The results are given in Table 9 and FIG. 18.

**TABLE 9**

| Yield(per 10⁶ cells) | MSC-Exo | IFNγ MSC-Exo |
|---|---|---|
| Exosomal RNA (ng) | 31.0 | 155.0 |

| | | |
|---|---|---|
| (MSC: non-treated mesenchymal stem cells, IFNγ MSC: IFNγ-treated mesenchymal stem cells) | | |

As can be seen in Table 9 and FIG. 18, the stem cell pretreatment substance [interferon-γ]-treated mesenchymal stem cells produced about 5.0-fold greater exosomal RNA than the non-treated mesenchymal stem cells.

From the results, it is understood that the content of exosomal RNA as well as the number of exosomes are increased by treatment of mesenchymal stem cells with the stem cell pretreatment substance according to the present disclosure.

### EXPERIMENT EXAMPLE 7. Functional Reinforcement of Stem Cells by Treatment with Tetrandrine

### 7-1. Increase of stem cell proliferation by treatment with tetrandrine

Mesenchymal stem cells and the tetrandrine - pretreated mesenchymal stem cells of the Example were each seeded at a density of 3,000 cells/well into 96-well plates containing 100 µL of a culture medium [high glucose DMEM (Gibco, Cat no.11995-065), 10% fetal bovine serum (HyClone), 1% MEM non-essential amino acids solution (100X) (Gibco, Cat no.11140-050)] per well and cultured for 24 hours in a CO₂ incubator. Then, CCK solution (10 µL/well) was added, and incubation was continued for 4 hours in the CO₂ incubator, followed by reading absorbance at a wavelength of 450 nm (420-480 nm) to determine proliferation.

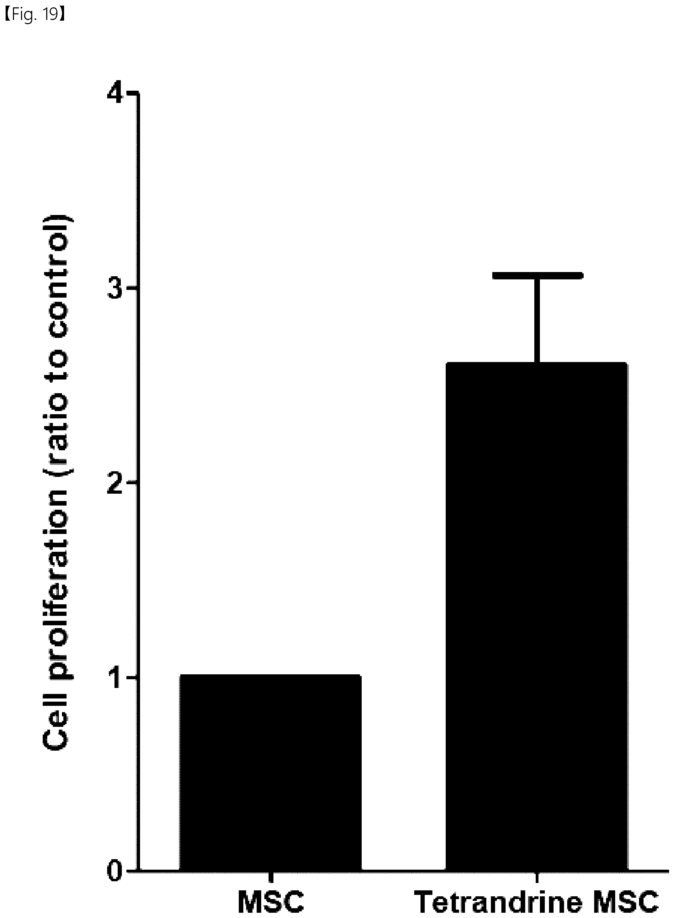

As can be seen in FIG. 19, the mesenchymal stem cells pretreated with the stem cell pretreatment substance [tetrandrine] increased in proliferation by about 260 %, with the ratio from 1 to 2.6 relative to the non-treated mesenchymal stem cells.

### 7-2. Increase of stem cell sternness by treatment with tetrandrine

Mesenchymal stem cells and the tetrandrine-pretreated mesenchymal stem cells of the Example were each seeded at a density of 1,000 cells/dish into 100-mm cell culture dishes containing 100 mL of a culture medium [high glucose DMEM (Gibco, Cat no.11995-065), 10% fetal bovine serum (HyClone), 1% MEM non-essential amino acids solution (100X) (Gibco, Cat no.11140-050)] per dish and cultured for 21 days. The cell-attached dishes were washed twice with PBS, followed by fixation with 95% methanol at room temperature for about 2 min. The fixed cells were washed three times with PBS and stained with 0.5% crystal violet [(sigma, C-3886, USA) 5g, methanol 100 ml] for 5 min. After water washing and drying at room temperature, colonies, each composed of 50 or more cells, were counted for comparison.

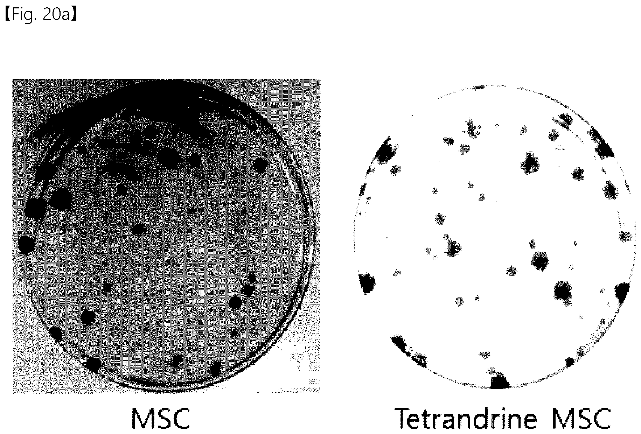

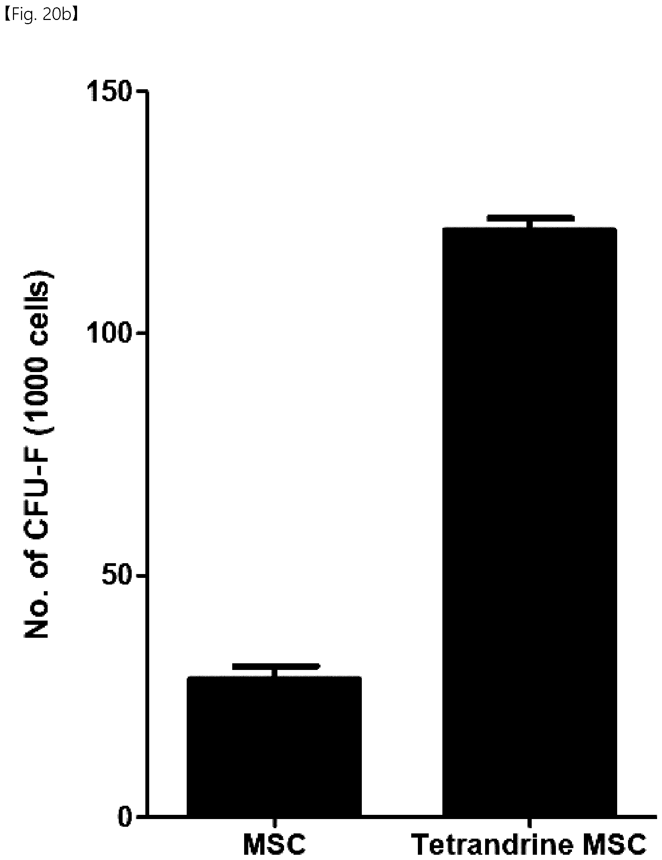

As can be seen in FIGS. 20a and 20b, more colonies were formed by the mesenchymal stem cells pretreated with the stem cell pretreatment substance [tetrandrine] (121.3 colonies) than the non-treated mesenchymal stem cells (28.7), with CFU-F increasing by about 423%.

### EXPERIMENTAL EXAMPLE 8. Increase of Exosome Productivity by Treatment with Tetrandrine

### 8-1. Increase in number of exosomes by treatment with tetrandrine

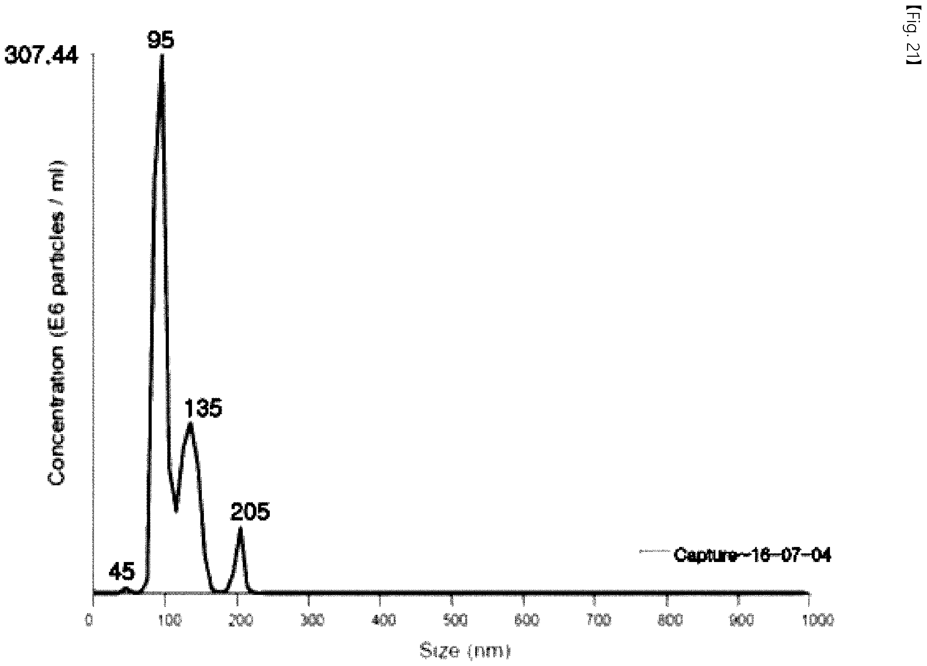

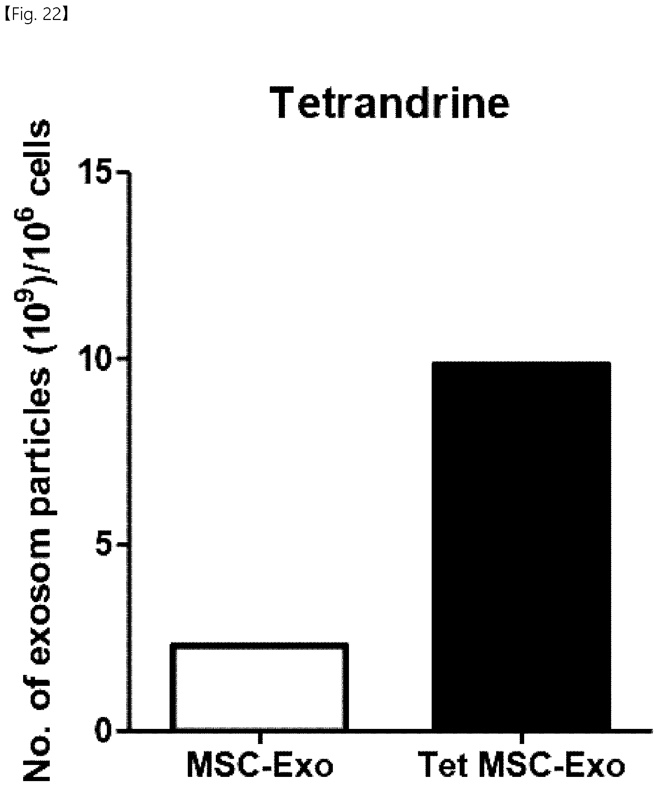

The exosomes isolated in the Example were counted the nanoparticle tracking assay (NanoSight NS300, Malvern). For comparison, exosomes were counted in the same manner with the exception that the cells were not pretreated with any pretreatment substance. The results are given in Table 10 and FIGS. 21 and 22.

**TABLE 10**

| Yield (per 10⁶ cells) | MSC-Exo | Tet MSC-Exo |
|---|---|---|
| No. of exosome particles (10⁹) | 2.3 | 9.84 |

| | | |
|---|---|---|
| (MSC: non-treated mesenchymal stem cells, Tet MSC: tetrandrine-treated mesenchymal stem cells) | | |

As can be seen in Table 10 and FIG. 22, the stem cell pretreatment substance [tetrandrine]-treated mesenchymal stem cells produced about 4.3-fold more exosomes than the non-treated mesenchymal stem cells.

From the results, it is understood that treatment of mesenchymal stem cells with the stem cell pretreatment substance according to the present disclosure increases the quantity of exosomes produced by the mesenchymal stem cells.

### 8-2. Increase in content of exosomal protein by treatment with tetrandrine

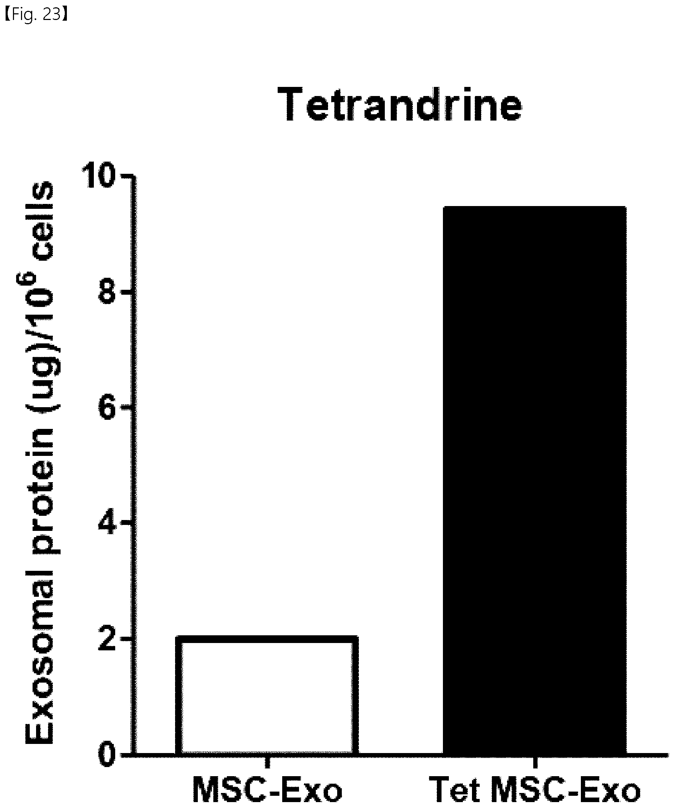

Proteins were isolated from the exosomes isolated in the Examples, using an exosome protein isolation kit (total exosome RNA and protein isolation kit, Invitrogen), and quantitatively measured by reading absorbance at 595 nm through Bradford analysis. For comparison, proteins were isolated from exosomes and quantitated in the same manner with the exception that the cells were not pretreated with any pretreatment substance. The results are given in Table 11 and FIG. 23.

**TABLE 11**

| Yield (per 10⁶ cells) | MSC-Exo | Tet MSC-Exo |
|---|---|---|
| Exosomal protein (ug) | 2.0 | 9.42 |

| | | |
|---|---|---|
| (MSC: non-treated mesenchymal stem cells, Tet MSC: Tetrandrine-treated mesenchymal stem cells) | | |

As can be seen in Table 11 and FIG. 23, the stem cell pretreatment substance [tetrandrine]-treated mesenchymal stem cells produced about 4.5-fold greater exosomal proteins than the non-treated mesenchymal stem cells.

From the results, it is understood that the content of exosomal proteins as well as the number of exosomes are increased by treatment of mesenchymal stem cells with the stem cell pretreatment substance according to the present disclosure.

### 8-3. Increase in content of exosomal RNA by treatment with tetrandrine

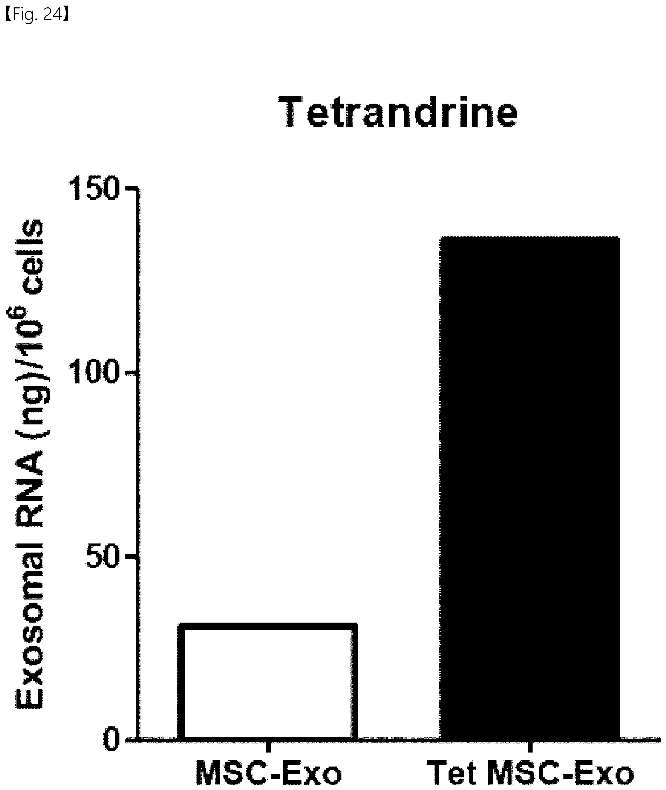

Using an RNA isolation kit (total exosome RNA and protein isolation kit, Invitrogen), total exosomal RNA was isolated from the exosomes isolated in the Example, followed by measuring RNA concentrations with the aid of a NanoDrop spectrometer. For comparison, total exosomal RNA was isolated from exosomes and quantitated in the same manner with the exception that the cells were not pretreated with any pretreatment substance. The results are given in Table 12 and FIG. 24.

**TABLE 12**

| Yield(per 10⁶ Cells) | MSC-Exo | Tet MSC-Exo |
|---|---|---|
| Exosomal RNA(ng) | 31.0 | 136.0 |

| | | |
|---|---|---|
| (MSC: non-treated mesenchymal stem cells, Tet MSC: Tetrandrine-treated mesenchymal stem cells) | | |

As can be seen in Table 12 and FIG. 24, the stem cell pretreatment substance [tetrandrine]-treated mesenchymal stem cells produced about 4.4-fold greater exosomal RNA than the non-treated mesenchymal stem cells.

From the results, it is understood that the content of exosomal RNA as well as the number of exosomes are increased by treatment of mesenchymal stem cells with the stem cell pretreatment substance according to the present disclosure.

### EXPERIMENT EXAMPLE 9. Functional Reinforcement of Stem Cells by Treatment with Hyaluronic acid

### 9-1. Increase of stem cell proliferation by treatment with hyaluronic acid

Mesenchymal stem cells and the hyaluronic acid-pretreated mesenchymal stem cells of the Example were each seeded at a density of 3,000 cells/well into 96-well plates containing 100 µL of a culture medium [high glucose DMEM (Gibco, Cat no.11995-065), 10% fetal bovine serum (HyClone), 1% MEM non-essential amino acids solution (100X) (Gibco, Cat no.11140-050)] per well and cultured for 24 hours in a CO₂ incubator. Then, CCK solution (10 µL/well) was added, and incubation was continued for 4 hours in the CO₂ incubator, followed by reading absorbance at a wavelength of 450 nm (420-480 nm) to determine proliferation.

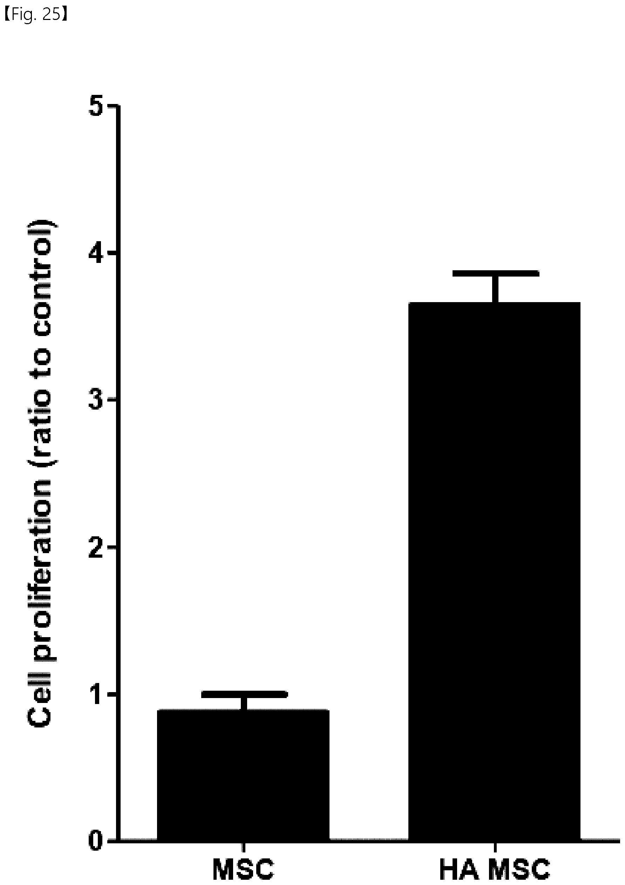

As can be seen in FIG. 25, the mesenchymal stem cells pretreated with the stem cell pretreatment substance [hyaluronic acid] increased in proliferation by about 364 %, with the ratio from 1 to 3.64 relative to the non-treated mesenchymal stem cells.

### 9-2. Increase of stem cell sternness by treatment with hyaluronic acid

Mesenchymal stem cells and the hyaluronic acid-pretreated mesenchymal stem cells of the Example were each seeded at a density of 1,000 cells/dish into 100-mm cell culture dishes containing 100 mL of a culture medium [high glucose DMEM (Gibco, Cat no.11995-065), 10% fetal bovine serum (HyClone), 1% MEM non-essential amino acids solution (100X) (Gibco, Cat no.11140-050)] per dish and cultured for 21 days. The cell-attached dishes were washed twice with PBS, followed by fixation with 95% methanol at room temperature for about 2 min. The fixed cells were washed three times with PBS and stained with 0.5% crystal violet [(sigma, C-3886, USA) 5g, methanol 100 ml] for 5 min. After water washing and drying at room temperature, colonies, each composed of 50 or more cells, were counted for comparison.

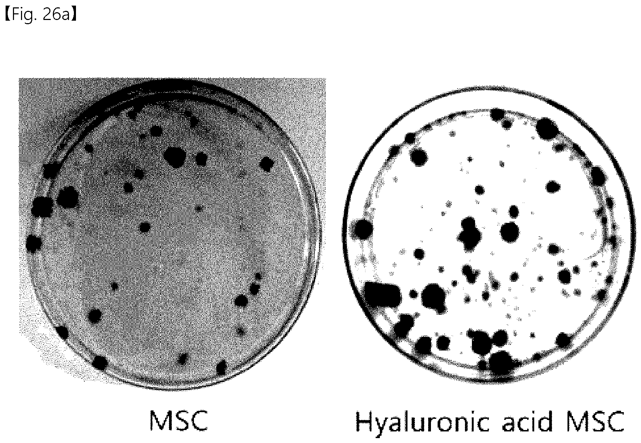

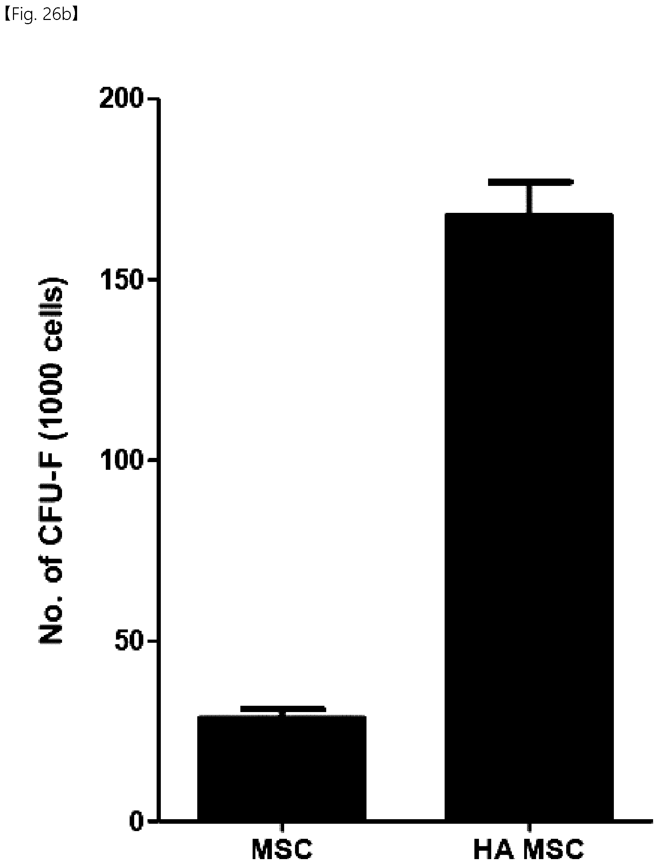

As can be seen in FIGS. 26a and 26b, more colonies were formed by the mesenchymal stem cells pretreated with the stem cell pretreatment substance [hyaluronic acid] (167.6 colonies) than the non-treated mesenchymal stem cells (28.6), with CFU-F increasing by about 586%.

### EXPERIMENTAL EXAMPLE 10. Increase of Exosome Productivity by Treatment with Hyaluronic acid

### 10-1. Increase in number of exosomes by treatment with hyaluronic acid

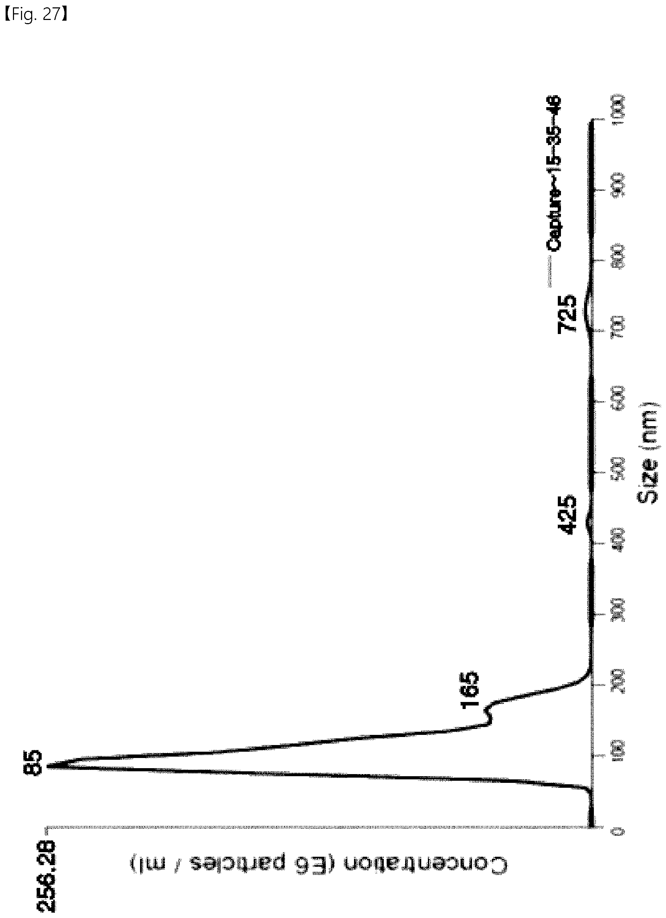

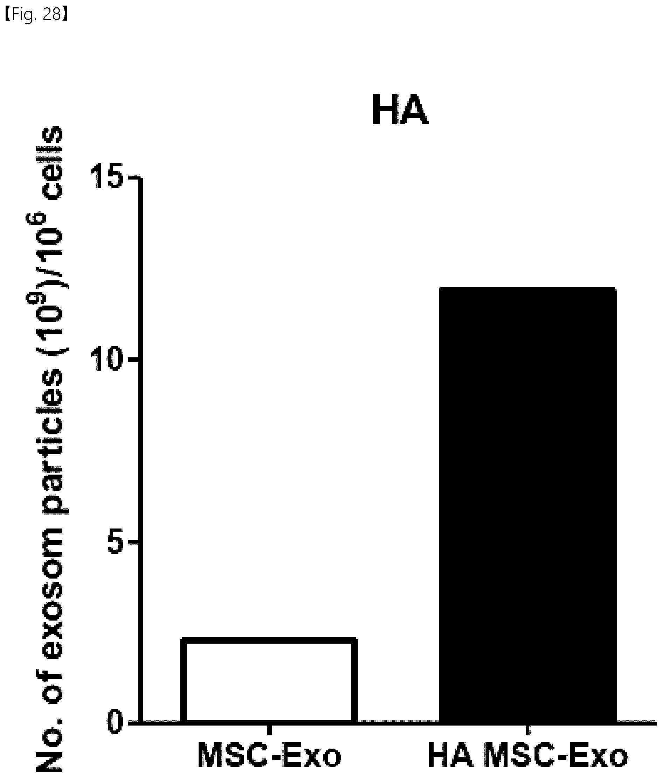

The exosomes isolated in the Example were counted the nanoparticle tracking assay (NanoSight NS300, Malvern). For comparison, exosomes were counted in the same manner with the exception that the cells were not pretreated with any pretreatment substance. The results are given in Table 13 and FIGS. 27 and 28.

**TABLE 13**

| Yield (per 10⁶ cells) | MSC-Exo | HA MSC-Exo |
|---|---|---|
| No. of exosome particles (10⁹) | 2.3 | 11.93 |

| | | |
|---|---|---|
| (MSC: non-treated mesenchymal stem cells, HA MSC: Hyaluronic acid-treated mesenchymal stem cells) | | |

As can be seen in Table 13 and FIG. 28, the stem cell pretreatment substance [hyaluronic acid]-treated mesenchymal stem cells produced about 5.2-fold more exosomes than the non-treated mesenchymal stem cells.

From the results, it is understood that treatment of mesenchymal stem cells with the stem cell pretreatment substance according to the present disclosure increases the quantity of exosomes produced by the mesenchymal stem cells.

### 10-2. Increase in content of exosomal protein by treatment with hyaluronic acid

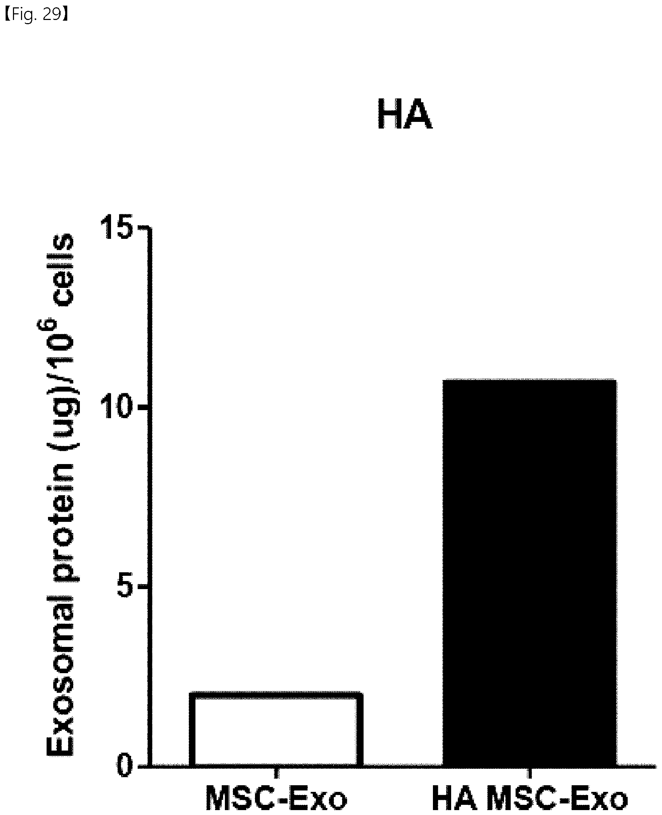

Proteins were isolated from the exosomes isolated in the Examples, using an exosome protein isolation kit (total exosome RNA and protein isolation kit, Invitrogen), and quantitatively measured by reading absorbance at 595 nm through Bradford analysis. For comparison, proteins were isolated from exosomes and quantitated in the same manner with the exception that the cells were not pretreated with any pretreatment substance. The results are given in Table 14 and FIG. 29.

**TABLE 14**

| Yield (per 10⁶ Cells) | MSC-Exo | HA MSC-Exo |
|---|---|---|
| Exosomal protein(ug) | 2.0 | 10.7 |

| | | |
|---|---|---|
| (MSC: non-treated mesenchymal stem cells, HA MSC: Hyaluronic acid- treated mesenchymal stem cells) | | |

As can be seen in Table 14 and FIG. 29, the stem cell pretreatment substance [hyaluronic acid]-treated mesenchymal stem cells produced about 5.35-fold greater exosomal proteins than the non-treated mesenchymal stem cells.

From the results, it is understood that the content of exosomal proteins as well as the number of exosomes are increased by treatment of mesenchymal stem cells with the stem cell pretreatment substance according to the present disclosure.

### 10-3. Increase in content of exosomal RNA by treatment with hyaluronic acid

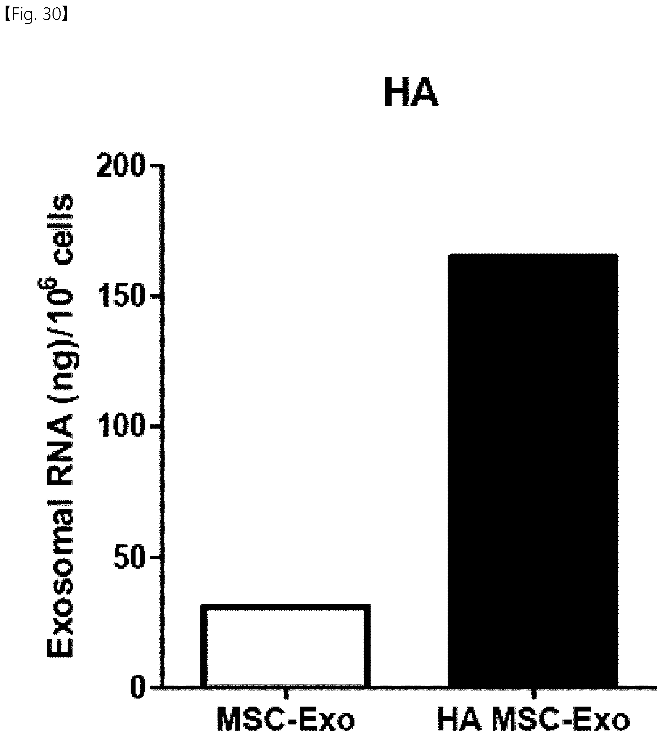

Using an RNA isolation kit (total exosome RNA and protein isolation kit, Invitrogen), total exosomal RNA was isolated from the exosomes isolated in the Example, followed by measuring RNA concentrations with the aid of a NanoDrop spectrometer. For comparison, total exosomal RNA was isolated from exosomes and quantitated in the same manner with the exception that the cells were not pretreated with any pretreatment substance. The results are given in Table 15 and FIG. 30.

**TABLE 15**

| Yield(per 10⁶ Cells) | MSC-Exo | HA MSC-Exo |
|---|---|---|
| Exosomal RNA(ng) | 31.0 | 165.0 |

| | | |
|---|---|---|
| (MSC: non-treated mesenchymal stem cells, HA MSC: Hyaluronic acid- treated mesenchymal stem cells) | | |

As can be seen in Table 15 and FIG. 30, the stem cell pretreatment substance [hyaluronic acid]-treated mesenchymal stem cells produced about 5.3-fold greater exosomal RNA than the non-treated mesenchymal stem cells.

From the results, it is understood that the content of exosomal RNA as well as the number of exosomes are increased by treatment of mesenchymal stem cells with the stem cell pretreatment substance according to the present disclosure.

### EXPERIMENT EXAMPLE 11. Functional Reinforcement of Stem Cells by Treatment with Substance P

### 11-1. Increase of stem cell proliferation by treatment with substance P

Mesenchymal stem cells and the substance P-pretreated mesenchymal stem cells of the Example were each seeded at a density of 3,000 cells/well into 96-well plates containing 100 µL of a culture medium [high glucose DMEM (Gibco, Cat no.11995-065), 10% fetal bovine serum (HyClone), 1% MEM non-essential amino acids solution (100X) (Gibco, Cat no.11140-050)] per well and cultured for 24 hours in a CO₂ incubator. Then, CCK solution (10 µL/well) was added, and incubation was continued for 4 hours in the CO₂ incubator, followed by reading absorbance at a wavelength of 450 nm (420-480 nm) to determine proliferation.

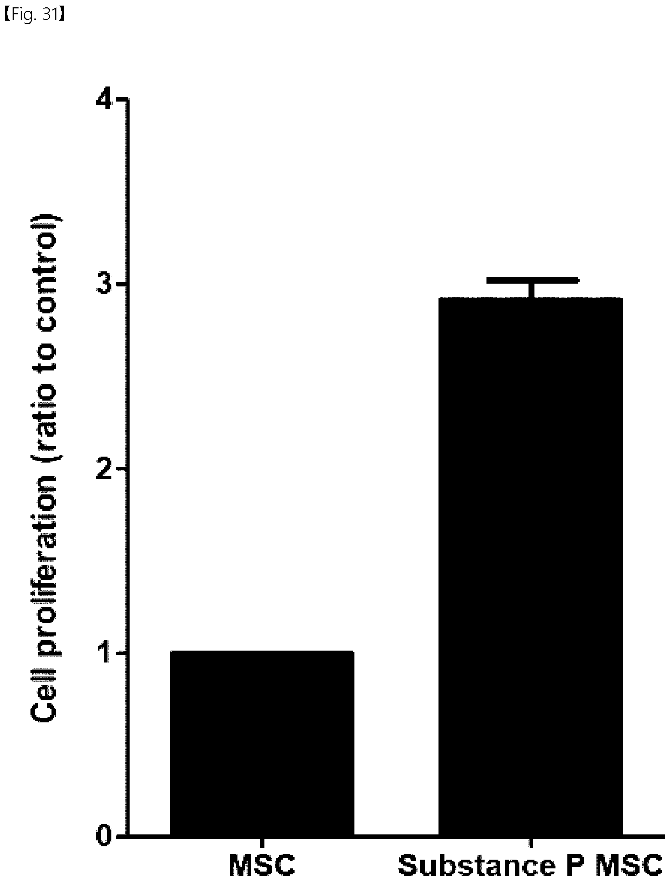

As can be seen in FIG. 31, the mesenchymal stem cells pretreated with the stem cell pretreatment substance [substance P] increased in proliferation by about 290 %, with the ratio from 1 to 2.9 relative to the non-treated mesenchymal stem cells.

### 11-2. Increase of stem cell sternness by treatment with substance P

Mesenchymal stem cells and the substance P-pretreated mesenchymal stem cells of the Example were each seeded at a density of 1,000 cells/dish into 100-mm cell culture dishes containing 100 mL of a culture medium [high glucose DMEM (Gibco, Cat no.11995-065), 10% fetal bovine serum (HyClone), 1% MEM non-essential amino acids solution (100X) (Gibco, Cat no.11140-050)] per dish and cultured for 21 days. The cell-attached dishes were washed twice with PBS, followed by fixation with 95% methanol at room temperature for about 2 min. The fixed cells were washed three times with PBS and stained with 0.5% crystal violet [(sigma, C-3886, USA) 5g, methanol 100 ml] for 5 min. After water washing and drying at room temperature, colonies, each composed of 50 or more cells, were counted for comparison.

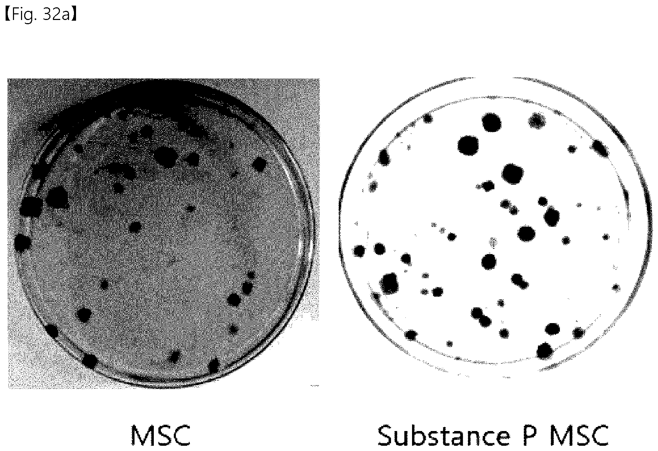

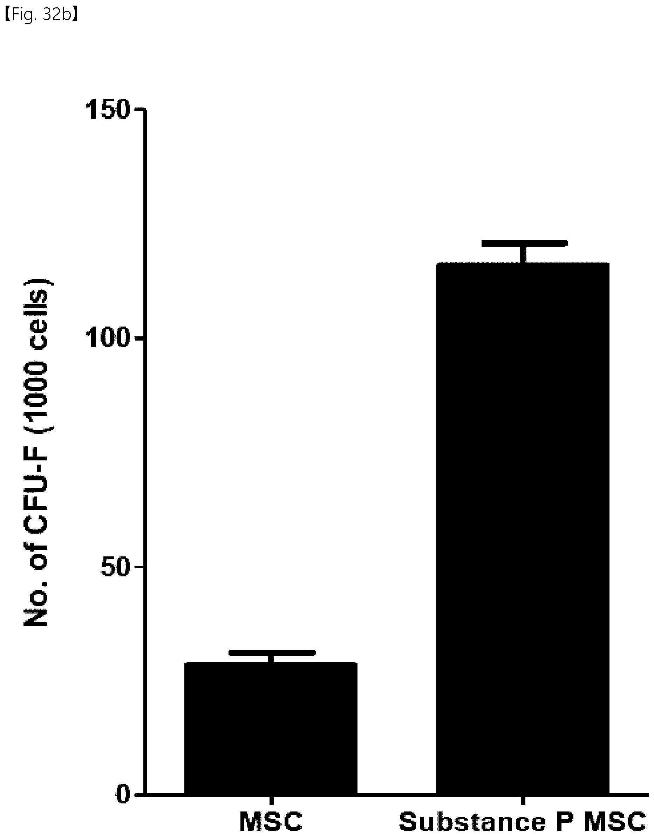

As can be seen in FIGS. 32a and 32b, more colonies were formed by the mesenchymal stem cells pretreated with the stem cell pretreatment substance [substance P] (116 colonies) than the non-treated mesenchymal stem cells (28.7), with CFU-F increasing by about 404%.

### EXPERIMENTAL EXAMPLE 12. Increase of Exosome Productivity by Treatment with Substance P

### 12-1. Increase in number of exosomes by treatment with substance P

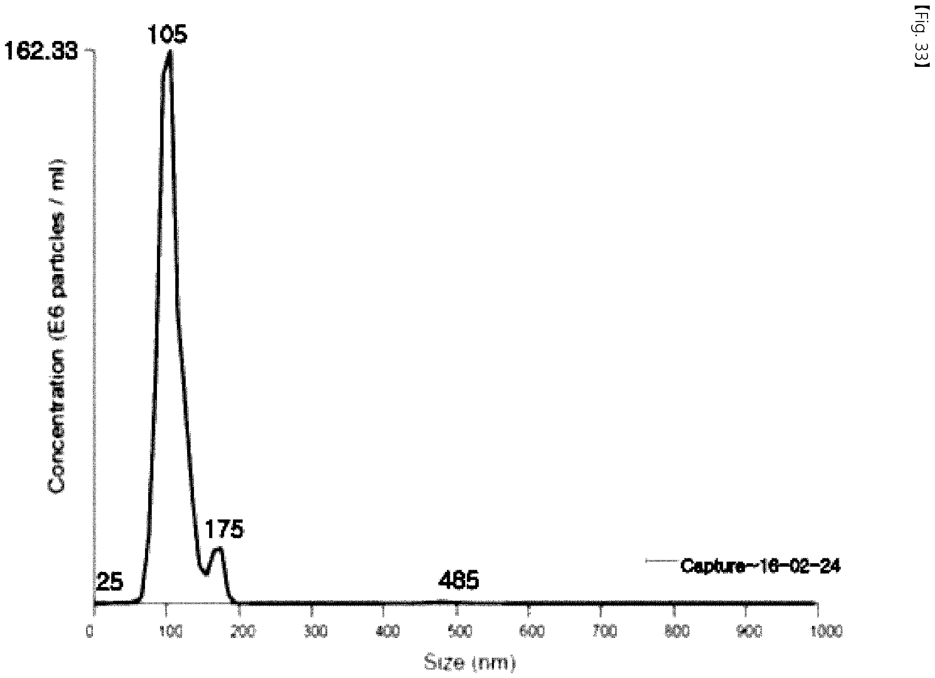

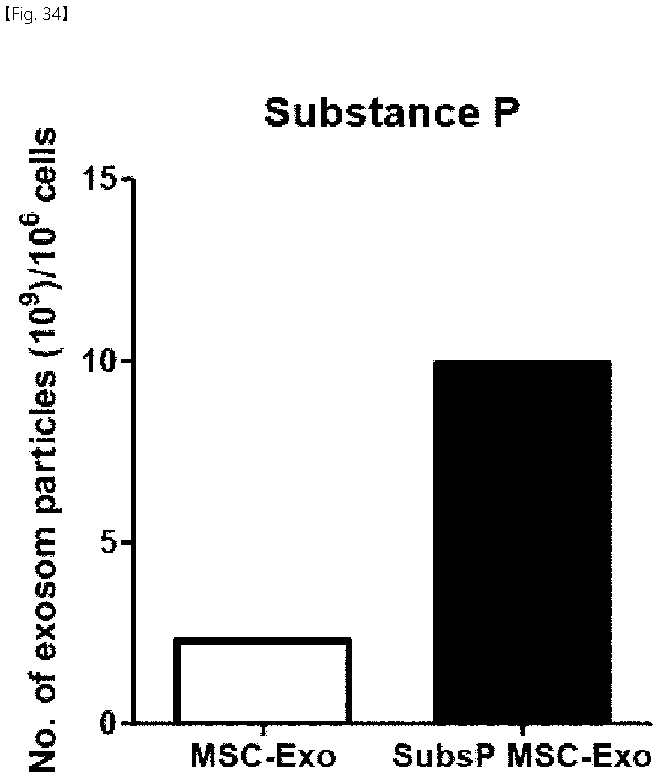

The exosomes isolated in the Example were counted the nanoparticle tracking assay (NanoSight NS300, Malvern). For comparison, exosomes were counted in the same manner with the exception that the cells were not pretreated with any pretreatment substance. The results are given in Table 16 and FIGS. 33 and 34.

**TABLE 16**

| Yield (per 10⁶ cells) | MSC-Exo | SubsP MSC-Exo |
|---|---|---|
| No. of exosome particles (10⁹) | 2.3 | 9.93 |

| | | |
|---|---|---|
| (MSC: non-treated mesenchymal stem cells, SubsP MSC: Substance P-treated mesenchymal stem cells) | | |

As can be seen in Table 16 and FIG. 34, the stem cell pretreatment substance [substance P]-treated mesenchymal stem cells produced about 4.3-fold more exosomes than the non-treated mesenchymal stem cells.

From the results, it is understood that treatment of mesenchymal stem cells with the stem cell pretreatment substance according to the present disclosure increases the quantity of exosomes produced by the mesenchymal stem cells.

### 12-2. Increase in content of exosomal protein by treatment with substance P

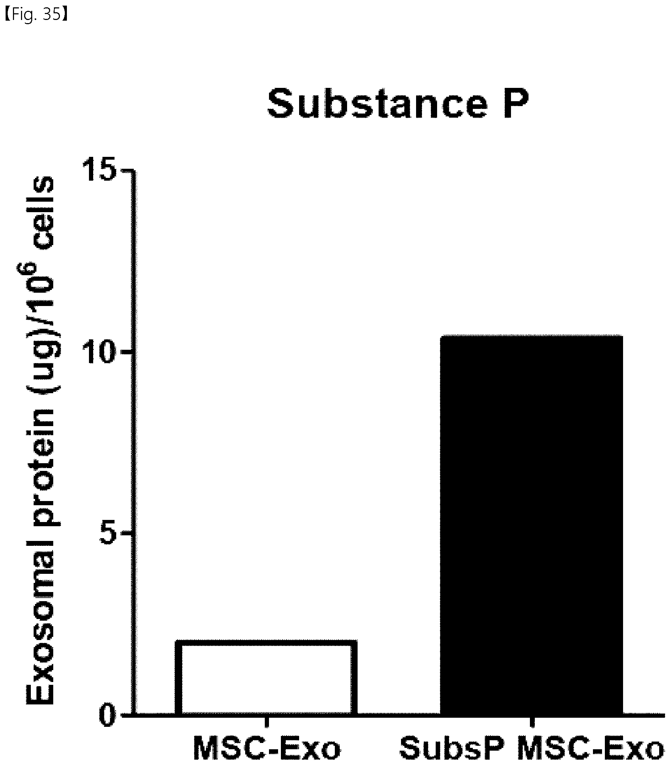

Proteins were isolated from the exosomes isolated in the Examples, using an exosome protein isolation kit (total exosome RNA and protein isolation kit, Invitrogen), and quantitatively measured by reading absorbance at 595 nm through Bradford analysis. For comparison, proteins were isolated from exosomes and quantitated in the same manner with the exception that the cells were not pretreated with any pretreatment substance. The results are given in Table 17 and FIG. 35.

**TABLE 17**

| Yield(per 10⁶ Cells) | MSC-Exo | SubsP MSC-Exo |
|---|---|---|
| Exosomal protein(ug) | 2.0 | 10.37 |

| | | |
|---|---|---|
| (MSC: non-treated mesenchymal stem cells, SubsP MSC: Substance P- treated mesenchymal stem cells) | | |

As can be seen in Table 17 and FIG. 35, the stem cell pretreatment substance [substance P]-treated mesenchymal stem cells produced about 5.2-fold greater exosomal proteins than the non-treated mesenchymal stem cells.

From the results, it is understood that the content of exosomal proteins as well as the number of exosomes are increased by treatment of mesenchymal stem cells with the stem cell pretreatment substance according to the present disclosure.

### 12-3. Increase in content of exosomal RNA by treatment with substance P

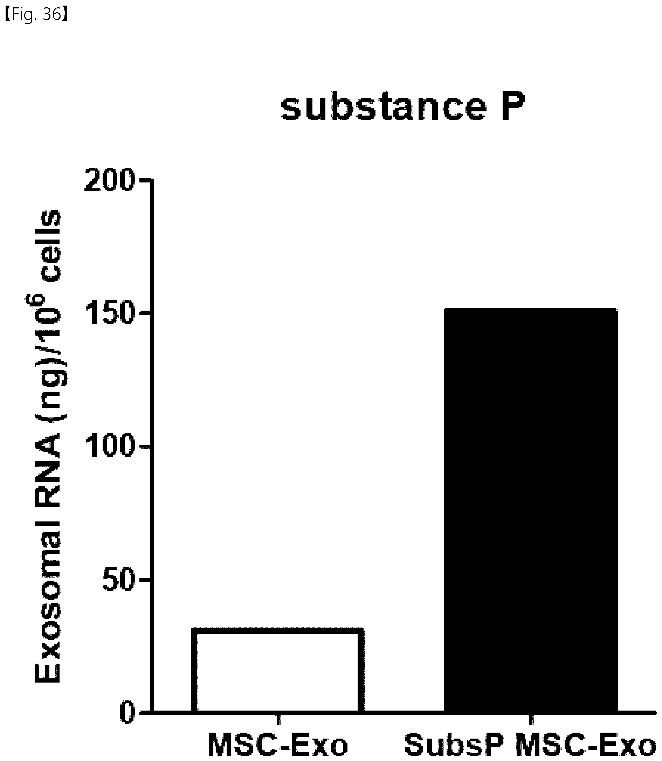

Using an RNA isolation kit (total exosome RNA and protein isolation kit, Invitrogen), total exosomal RNA was isolated from the exosomes isolated in the Example, followed by measuring RNA concentrations with the aid of a NanoDrop spectrometer. For comparison, total exosomal RNA was isolated from exosomes and quantitated in the same manner with the exception that the cells were not pretreated with any pretreatment substance. The results are given in Table 18 and FIG. 36.

**TABLE 18**

| Yield(per 10⁶ Cells) | MSC-Exo | SubsP MSC-Exo |
|---|---|---|
| Exosomal RNA(ng) | 31.0 | 150.84 |

| | | |
|---|---|---|
| (MSC: non-treated mesenchymal stem cells, SubsP MSC: Substance P- treated mesenchymal stem cells) | | |

As can be seen in Table 18 and FIG. 36, the stem cell pretreatment substance [substance P]-treated mesenchymal stem cells produced about 4.9-fold greater exosomal RNA than the non-treated mesenchymal stem cells.

From the results, it is understood that the content of exosomal RNA as well as the number of exosomes are increased by treatment of mesenchymal stem cells with the stem cell pretreatment substance according to the present disclosure.

### EXPERIMENT EXAMPLE 13. Functional Reinforcement of Stem Cells by Treatment with Resveratrol

### 13-1. Increase of stem cell proliferation by treatment with resveratrol

Mesenchymal stem cells and the resveratrol-pretreated mesenchymal stem cells of the Example were each seeded at a density of 3,000 cells/well into 96-well plates containing 100 µL of a culture medium [high glucose DMEM (Gibco, Cat no.11995-065), 10% fetal bovine serum (HyClone), 1% MEM non-essential amino acids solution (100X) (Gibco, Cat no.11140-050)] per well and cultured for 24 hours in a CO₂ incubator. Then, CCK solution (10 µL/well) was added, and incubation was continued for 4 hours in the CO₂ incubator, followed by reading absorbance at a wavelength of 450 nm (420-480 nm) to determine proliferation.

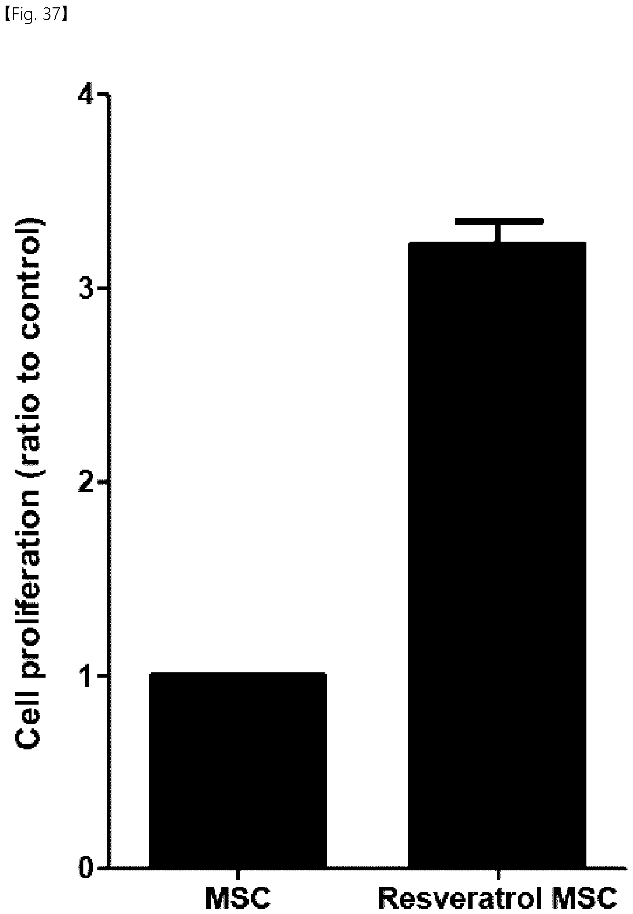

As can be seen in FIG. 37, the mesenchymal stem cells pretreated with the stem cell pretreatment substance [resveratrol] increased in proliferation by about 322 %, with the ratio from 1 to 3.22 relative to the non-treated mesenchymal stem cells.

### 13-2. Increase of stem cell sternness by treatment with resveratrol

Mesenchymal stem cells and the resveratrol-pretreated mesenchymal stem cells of the Example were each seeded at a density of 1,000 cells/dish into 100-mm cell culture dishes containing 100 mL of a culture medium [high glucose DMEM (Gibco, Cat no.11995-065), 10% fetal bovine serum (HyClone), 1% MEM non-essential amino acids solution (100X) (Gibco, Cat no.11140-050)] per dish and cultured for 21 days. The cell-attached dishes were washed twice with PBS, followed by fixation with 95% methanol at room temperature for about 2 min. The fixed cells were washed three times with PBS and stained with 0.5% crystal violet [(sigma, C-3886, USA) 5g, methanol 100 ml] for 5 min. After water washing and drying at room temperature, colonies, each composed of 50 or more cells, were counted for comparison.

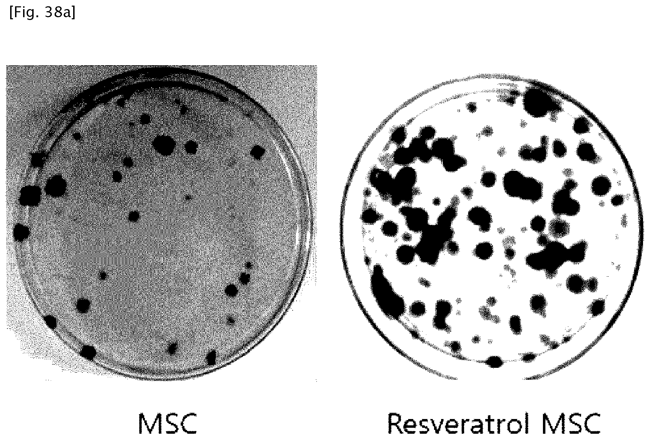

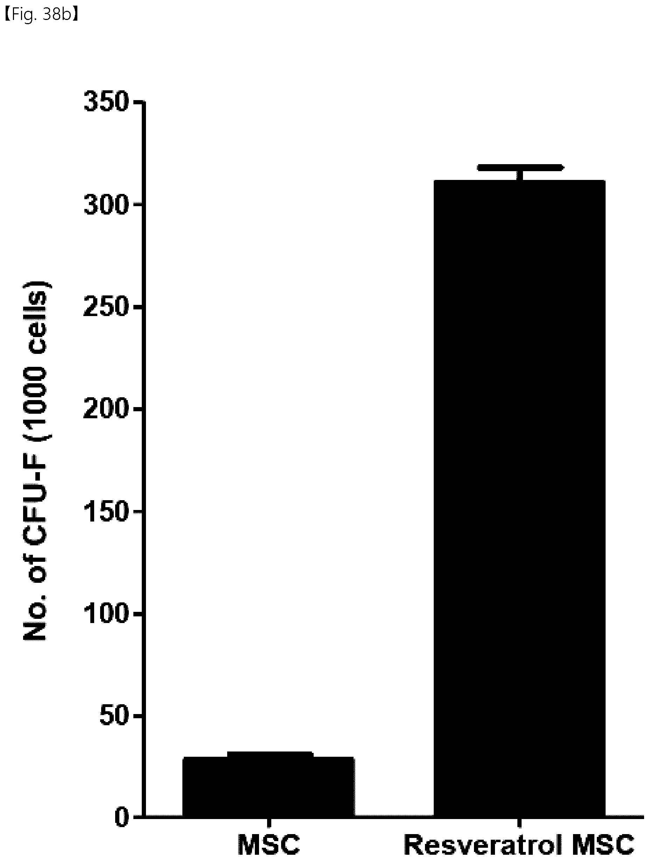

As can be seen in FIGS. 38a and 38b, more colonies were formed by the mesenchymal stem cells pretreated with the stem cell pretreatment substance [resveratrol] (311 colonies) than the non-treated mesenchymal stem cells (28.6), with CFU-F increasing by about 1,087%.

### EXPERIMENTAL EXAMPLE 14. Increase of Exosome Productivity by Treatment with Resveratrol

### 14-1. Increase in number of exosomes by treatment with resveratrol

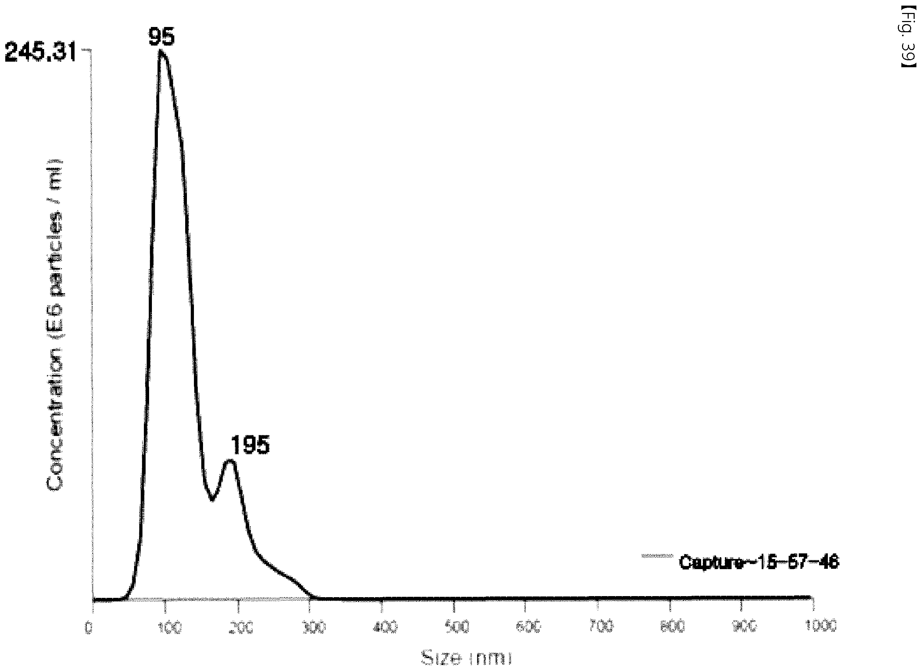

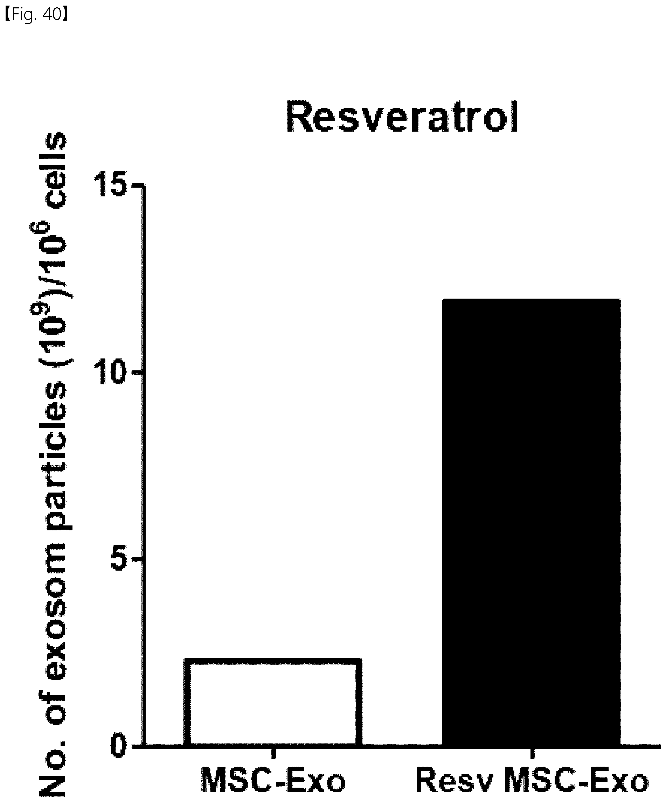

The exosomes isolated in the Example were counted the nanoparticle tracking assay (NanoSight NS300, Malvern). For comparison, exosomes were counted in the same manner with the exception that the cells were not pretreated with any pretreatment substance. The results are given in Table 19 and FIGS. 39 and 40.

**TABLE 19**

| Yield (per 10⁶ cells) | MSC-Exo | Resv MSC-Exo |
|---|---|---|
| No. of exosome particles (10⁹) | 2.3 | 11. 9 |

| | | |
|---|---|---|
| (MSC: non-treated mesenchymal stem cells, Resv MSC: Resveratrol-treated mesenchymal stem cells) | | |

As can be seen in Table 19 and FIG. 40, the stem cell pretreatment substance [resveratrol]-treated mesenchymal stem cells produced about 5.2-fold more exosomes than the non-treated mesenchymal stem cells.

From the results, it is understood that treatment of mesenchymal stem cells with the stem cell pretreatment substance according to the present disclosure increases the quantity of exosomes produced by the mesenchymal stem cells.

### 14-2. Increase in content of exosomal protein by treatment with resveratrol

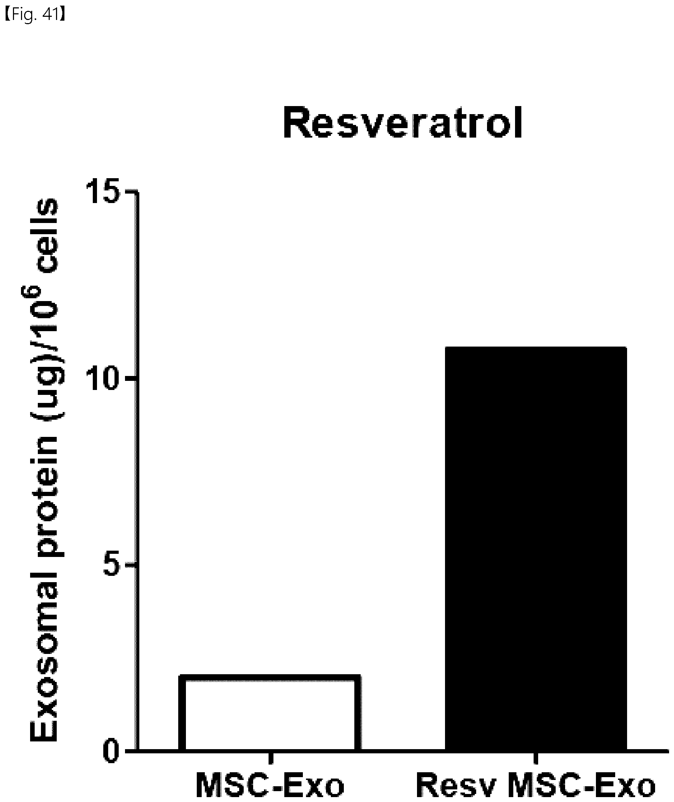

Proteins were isolated from the exosomes isolated in the Examples, using an exosome protein isolation kit (total exosome RNA and protein isolation kit, Invitrogen), and quantitatively measured by reading absorbance at 595 nm through Bradford analysis. For comparison, proteins were isolated from exosomes and quantitated in the same manner with the exception that the cells were not pretreated with any pretreatment substance. The results are given in Table 20 and FIG. 41.

**TABLE 20**

| Yield (per 10⁶ cells) | MSC-Exo | Resv MSC-Exo |
|---|---|---|
| Exosomal protein(ug) | 2.0 | 10.78 |

| | | |
|---|---|---|
| (MSC: non-treated mesenchymal stem cells, Resv MSC: Resveratrol-treated mesenchymal stem cells) | | |

As can be seen in Table 20 and FIG. 41, the stem cell pretreatment substance [resveratrol]-treated mesenchymal stem cells produced about 5.4-fold greater exosomal proteins than the non-treated mesenchymal stem cells.

From the results, it is understood that the content of exosomal proteins as well as the number of exosomes are increased by treatment of mesenchymal stem cells with the stem cell pretreatment substance according to the present disclosure.

### 14-3. Increase in content of exosomal RNA by treatment with resveratrol

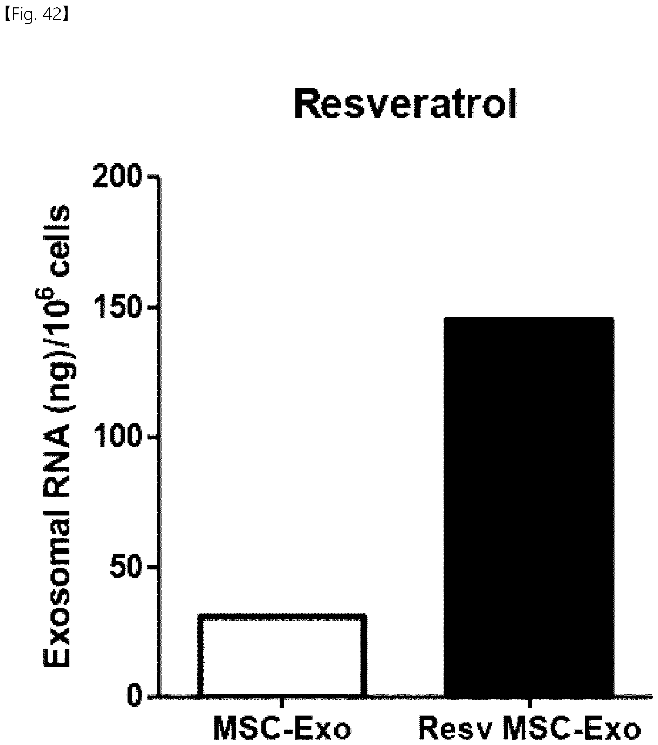

Using an RNA isolation kit (total exosome RNA and protein isolation kit, Invitrogen), total exosomal RNA was isolated from the exosomes isolated in the Example, followed by measuring RNA concentrations with the aid of a NanoDrop spectrometer. For comparison, total exosomal RNA was isolated from exosomes and quantitated in the same manner with the exception that the cells were not pretreated with any pretreatment substance. The results are given in Table 21 and FIG. 42.

**TABLE 21**

| Yield (per 10⁶ cells) | MSC-Exo | Resv MSC-Exo |
|---|---|---|
| Exosomal RNA (ng) | 31.0 | 145.15 |

| | | |
|---|---|---|
| (MSC: non-treated mesenchymal stem cells, Resv MSC: Resveratrol-treated mesenchymal stem cells) | | |

As can be seen in Table 21 and FIG. 42, the stem cell pretreatment substance [resveratrol]-treated mesenchymal stem cells produced about 4.7-fold greater exosomal RNA than the non-treated mesenchymal stem cells.

From the results, it is understood that the content of exosomal RNA as well as the number of exosomes are increased by treatment of mesenchymal stem cells with the stem cell pretreatment substance according to the present disclosure.

### EXPERIMENT EXAMPLE 15. Functional Reinforcement of Stem Cells by Treatment with Lanifibranor

### 15-1. Increase of stem cell proliferation by treatment with lanifibranor

Mesenchymal stem cells and the lanifibranor-pretreated mesenchymal stem cells of the Example were each seeded at a density of 3,000 cells/well into 96-well plates containing 100 µL of a culture medium [high glucose DMEM (Gibco, Cat no.11995-065), 10% fetal bovine serum (HyClone), 1% MEM non-essential amino acids solution (100X) (Gibco, Cat no.11140-050)] per well and cultured for 24 hours in a CO₂ incubator. Then, CCK solution (10 µL/well) was added, and incubation was continued for 4 hours in the CO₂ incubator, followed by reading absorbance at a wavelength of 450 nm (420-480 nm) to determine proliferation.

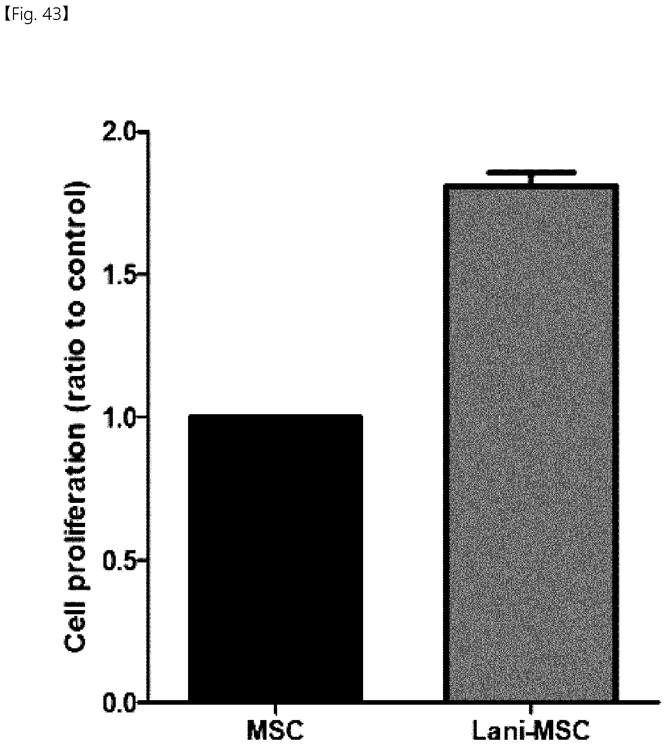

As can be seen in FIG. 43, the mesenchymal stem cells pretreated with the stem cell pretreatment substance [lanifibranor] increased in proliferation by about 181 %, with the ratio from 1 to 1.81 relative to the non-treated mesenchymal stem cells.

### 15-2. Increase of stem cell sternness by treatment with lanifibranor

Mesenchymal stem cells and the lanifibranor-pretreated mesenchymal stem cells of the Example were each seeded at a density of 1,000 cells/dish into 100-mm cell culture dishes containing 100 mL of a culture medium [high glucose DMEM (Gibco, Cat no.11995-065), 10% fetal bovine serum (HyClone), 1% MEM non-essential amino acids solution (100X) (Gibco, Cat no.11140-050)] per dish and cultured for 21 days. The cell-attached dishes were washed twice with PBS, followed by fixation with 95% methanol at room temperature for about 2 min. The fixed cells were washed three times with PBS and stained with 0.5% crystal violet [(sigma, C-3886, USA) 5g, methanol 100 ml] for 5 min. After water washing and drying at room temperature, colonies, each composed of 50 or more cells, were counted for comparison.

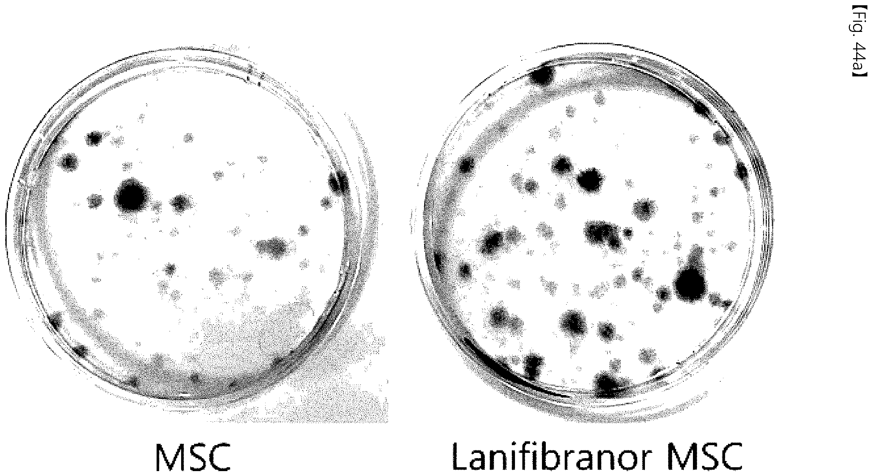

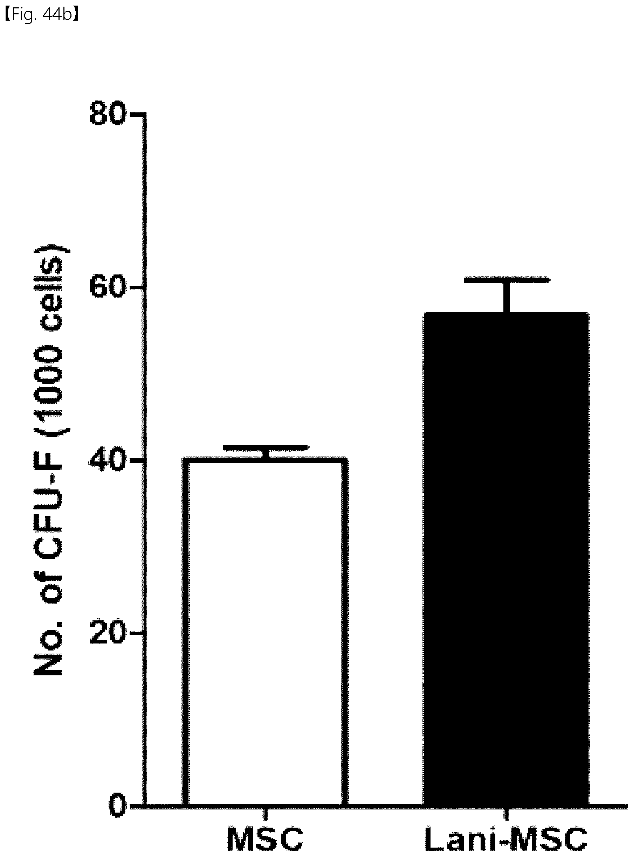

As can be seen in FIGS. 44a and 44b, more colonies were formed by the mesenchymal stem cells pretreated with the stem cell pretreatment substance [lanifibranor] (56.7 colonies) than the non-treated mesenchymal stem cells (40), with CFU-F increasing by about 142%.

### EXPERIMENTAL EXAMPLE 16. Increase of Exosome Productivity by Treatment with Lanifibranor

### 16-1. Increase in number of exosomes by treatment with lanifibranor

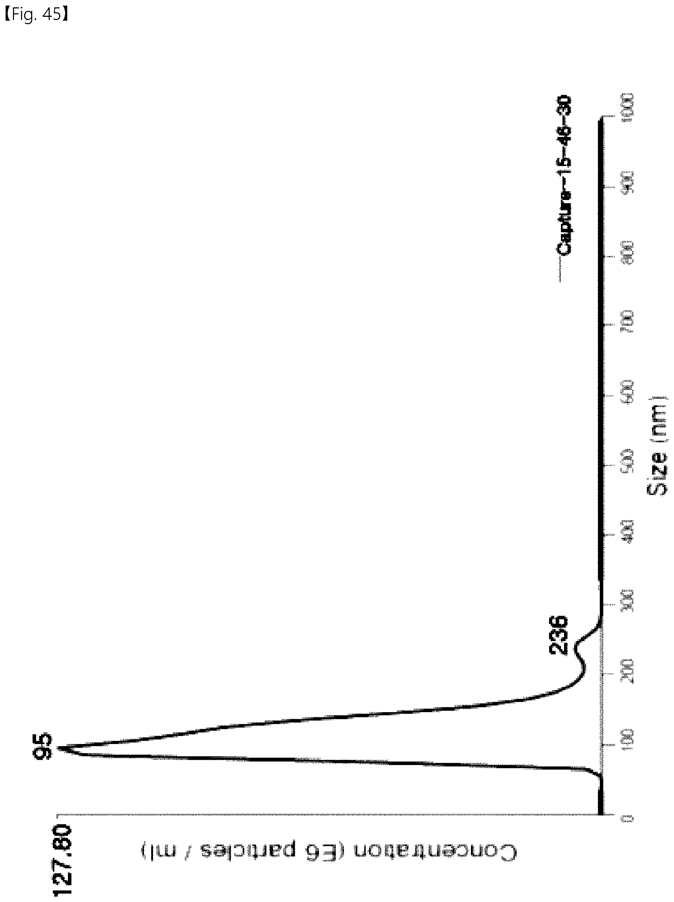

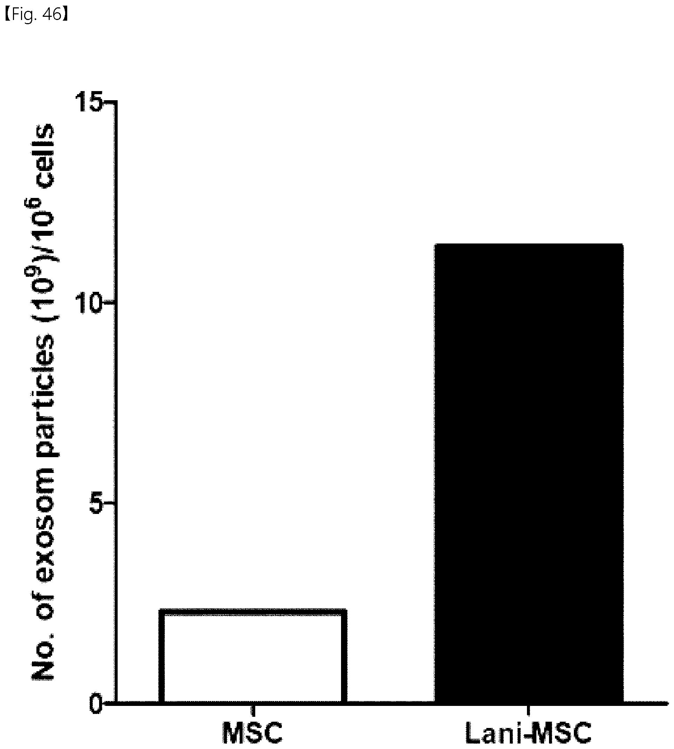

The exosomes isolated in the Example were counted the nanoparticle tracking assay (NanoSight NS300, Malvern). For comparison, exosomes were counted in the same manner with the exception that the cells were not pretreated with any pretreatment substance. The results are given in Table 22 and FIGS. 45 and 46.

**TABLE 22**

| Yield (per 10⁶ cells) | MSC-Exo | Lani-MSC-Exo |
|---|---|---|
| No. of exosome particles(10⁹) | 2.30 | 11.4 |

| | | |
|---|---|---|
| (MSC: non-treated mesenchymal stem cells, Lani-MSC-Exo: Lanifibranor-treated mesenchymal stem cells) | | |

As can be seen in Table 22 and FIG. 46, the stem cell pretreatment substance [lanifibranor]-treated mesenchymal stem cells produced about 4.95-fold more exosomes than the non-treated mesenchymal stem cells.

From the results, it is understood that treatment of mesenchymal stem cells with the stem cell pretreatment substance according to the present disclosure increases the quantity of exosomes produced by the mesenchymal stem cells.

### 16-2. Increase in content of exosomal protein by treatment with lanifibranor

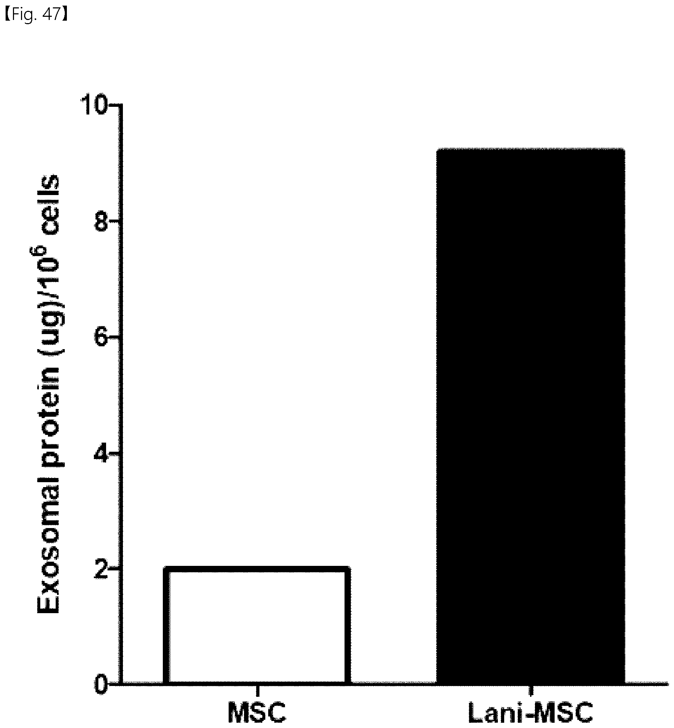

Proteins were isolated from the exosomes isolated in the Examples, using an exosome protein isolation kit (total exosome RNA and protein isolation kit, Invitrogen), and quantitatively measured by reading absorbance at 595 nm through Bradford analysis. For comparison, proteins were isolated from exosomes and quantitated in the same manner with the exception that the cells were not pretreated with any pretreatment substance. The results are given in Table 23 and FIG. 47.

**TABLE 23**

| Yield(per 10⁶ Cells) | MSC-Exo | Lani-MSC-Exo |
|---|---|---|
| Exosomal protein(ug) | 2.0 | 9.2 |

| | | |
|---|---|---|
| (MSC: non-treated mesenchymal stem cells, Lani-MSC-Exo: Lanifibranor-treated mesenchymal stem cells) | | |

As can be seen in Table 23 and FIG. 47, the stem cell pretreatment substance [lanifibranor]-treated mesenchymal stem cells produced about 4.6-fold greater exosomal proteins than the non-treated mesenchymal stem cells.

From the results, it is understood that the content of exosomal proteins as well as the number of exosomes are increased by treatment of mesenchymal stem cells with the stem cell pretreatment substance according to the present disclosure.

### 16-3. Increase in content of exosomal RNA by treatment with lanifibranor

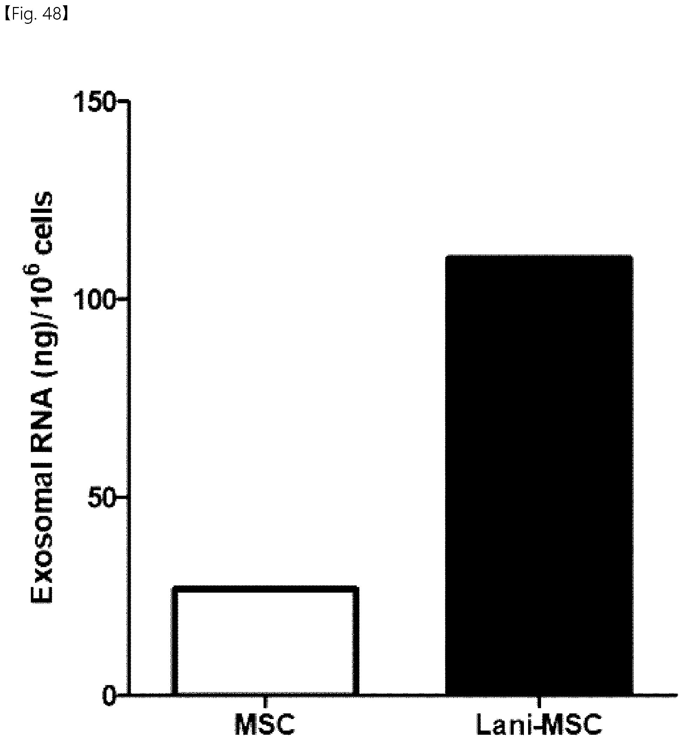

Using an RNA isolation kit (total exosome RNA and protein isolation kit, Invitrogen), total exosomal RNA was isolated from the exosomes isolated in the Example, followed by measuring RNA concentrations with the aid of a NanoDrop spectrometer. For comparison, total exosomal RNA was isolated from exosomes and quantitated in the same manner with the exception that the cells were not pretreated with any pretreatment substance. The results are given in Table 24 and FIG. 48.

**TABLE 24**

| Yield (per 10⁶ Cells) | MSC-Exo | Lani-MSC-Exo |
|---|---|---|
| Exosomal RNA(ng) | 27.00 | 110.34 |

| | | |
|---|---|---|
| (MSC: non-treated mesenchymal stem cells, Lani-MSC-Exo: Lanifibranor- treated mesenchymal stem cells) | | |

As can be seen in Table 24 and FIG. 48, the stem cell pretreatment substance [lanifibranor]-treated mesenchymal stem cells produced about 4.1-fold greater exosomal RNA than the non-treated mesenchymal stem cells.

From the results, it is understood that the content of exosomal RNA as well as the number of exosomes are increased by treatment of mesenchymal stem cells with the stem cell pretreatment substance according to the present disclosure.

### Conclusion

When treated with the stem cell pretreatment substance (exendin-4, phorbol 12-myristate 13-acetate (PMA), interferon-γ, tetrandrine, hyaluronic acid, substance P, resveratrol, or lanifibranor) according to the present disclosure, mesenchymal stem cells were found to exhibit increased stemness. It was also found that the mesenchymal stem cells treated with the stem cell pretreatment substance according to the present disclosure increased in the productivity of exosomal protein and the content of exosomal RNA as well as producing an increased quantity of exosomes.

Therefore, the stem cell pretreatment substance (exendin-4, phorbol 12-myristate 13-acetate (PMA), interferon-γ, tetrandrine, hyaluronic acid, substance P, resveratrol, or lanifibranor) according to the present disclosure promotes the production of stem cell-derived exosomes and, as such, is expected to be used in the production of highly functional stem cells and the mass production of exosomes.

### Industrial Applicability

The present disclosure relates to a composition for promoting the production of stem cell-derived exosomes and increasing the stemness of stem cells.

## Claims

1. A composition for promoting production of exosomes derived from a stem cell, the composition comprising at least one selected from the group consisting of exendin-4, phorbol 12-myristate 13-acetate (PMA), interferon-γ, tetrandrine, hyaluronic acid, substance P, resveratrol, and lanifibranor.

2. The composition of claim 1, wherein the stem cell is an embryonic stem cell, an adult stem cell, or an induced pluripotent stem cell (iPSC).

3. The composition of claim 2, wherein the adult stem cell is an adult stem cell of human or animal tissue origin, a mesenchymal stromal cell of human or animal tissue origin, or an induced pluripotent stem cell of human or animal tissue origin.

4. The composition of claim 3, wherein the human or animal tissue is selected from the group consisting of umbilical cord, umbilical cord blood, lipid, muscle, nerve, skin, amnion, and placenta.

5. The composition of claim 3, wherein the adult stem cell of human or animal tissue origin is selected from the group consisting of a hematopoietic stem cell, a mammary stem cell, an intestinal stem cell, a vascular endothelial progenitor cell, a neural stem cell, an olfactory neural stem cell, and a testicular stem cell.

6. A composition for increasing stemness of stem cell, the composition comprising at least one selected from the group consisting of exendin-4, phorbol 12-myristate 13-acetate (PMA), interferon-γ, tetrandrine, hyaluronic acid, substance P, resveratrol, and lanifibranor.

7. A stem cell therapy product, comprising the composition of any one of claims 1 to 6.

8. A stem cell-derived exosome, wherein the stem cell is pretreated with at least one selected from the group consisting of exendin-4, phorbol 12-myristate 13-acetate (PMA), interferon-γ, tetrandrine, hyaluronic acid, substance P, resveratrol, and lanifibranor.

9. A stem cell therapy product, comprising the stem cell-derived exosome of claim 8 as an active ingredient.

10. A method for producing stem cell-derived exosomes, the method comprising a step of:
culturing stem cells in a cell culture medium containing at least one selected from the group consisting of exendin-4, phorbol 12-myristate 13-acetate (PMA), interferon-γ, tetrandrine, hyaluronic acid, substance P, resveratrol, and lanifibranor.

11. The method of claim 10, further comprising the steps of:
washing the cultured stem cells, followed by additional culturing in a cell culture medium; and
isolating exosomes.

12. The method of claim 10, wherein the cell culture medium contains fetal bovine serum free of exosomes.

13. A method for increasing stemness of stem cells, the method comprising a step of:
culturing stem cells in a cell culture medium containing at least one selected from the group consisting of exendin-4, phorbol 12-myristate 13-acetate (PMA), interferon-γ, tetrandrine, hyaluronic acid, substance P, resveratrol, and lanifibranor.
